# FASCICULE DE BREVET EUROPEEN

(11) **EP 1 049 721 B1**
(45) Date de publication et mention de la délivrance du brevet: **21.11.2001**
(21) Numéro de dépôt: 99900524.2
(22) Date de dépôt: 13.01.1999
(51) Int. Cl.: C08B 37/00, A61K 31/715

(54) **POLYSACCHARIDES DE SYNTHESE, PROCEDE POUR LEUR PREPARATION ET COMPOSITIONS PHARMACEUTIQUES LE CONTENANT**
SYNTHETISCHE POLYSACCHARIDE, VERFAHREN ZU DEREN HERSTELLUNG UND DIESE ENTHALTENDE PHARMAZEUTISCHE ZUSAMMENSETZUNGEN
SYNTHETIC POLYSACCHARIDES, THEIR METHOD OF PRODUCTION AND PHARMACEUTICAL COMPOSITIONS CONTAINING SAME

(30) Priorité: 19.01.1998 FR 9800515
(43) Date de publication de la demande: 08.11.2000
(73) Titulaire: SANOFI-SYNTHELABO, 75013 Paris (FR); Akzo Nobel N.V., 6824 BM Arnhem (NL)
(72) Inventeur: BASTEN, Johannes, NL-6654 AV Afferden (NL); DREEF-TROMP, Cornelia, NL-6605 SN Wijchen (NL); DRIGUEZ, Pierre-Alexandre, F-31500 Toulouse (FR); DUCHAUSSOY, Philippe, F-31300 Toulouse (FR); HERBERT, Jean-Marc, F-31170 Tournefeuille (FR); PETITOU, Maurice, F-75645 Paris Cedex 13 (FR); VAN BOECKEL, Constant, NL-5345 LX Oss (NL)
(74) Mandataire: Thouret-Lemaitre, Elisabeth
(86) Numéro de dépôt international: FR9900044
(87) Numéro de publication internationale: WO9936443

(56) Documents cités:
- WO-A-97/47659

## Description

La présente invention concerne des nouveaux polysaccharides de synthèse possédant les activités pharmacologiques anticoagulante et antithrombotique de l'héparine.

L'héparine appartient à la famille des glycosaminoglycanes (GAGs), qui sont des polysaccharides sulfatés naturels hétérogènes.

Les préparations d'héparine sont des mélanges de chaînes comprenant un nombre d'unités monosaccharidiques allant de 10 jusqu'à 100 et plus. À cette hétérogénéité de taille moléculaire s'ajoute une hétérogénéité de structure, au niveau de la nature des monosaccharides constitutifs, mais également au niveau des substituants qu'ils portent (L. Rodén in : « The Biochemistry of Glycoproteins and Glycosaminoglycans », Ed. by Lennarz W.J., Plenum Press, New York and London, 267-371, 1980).

Chaque famille de GAGs naturels possède en général un éventail d'activités pharmacologiques. Toutes sont réunies dans les préparations que l'on peut obtenir à partir de produits naturels. Ainsi, par exemple, les héparines et les héparanes sulfate possèdent une activité antithrombotique qui est liée à l'action simultanée sur plusieurs facteurs de la coagulation.

L'héparine catalyse, notamment *via* l'antithrombine III (AT III), l'inhibition de deux enzymes qui interviennent dans la cascade de la coagulation du sang à savoir, le facteur Xa et le facteur IIa (ou thrombine). Les préparations d'héparines de bas poids moléculaire (HBPM), contiennent des chaînes formées de 4 à 30 monosaccharides et ont la propriété d'agir plus sélectivement sur le facteur Xa que sur la thrombine.

Certains oligosaccharides de synthèse notamment ceux décrits dans EP 84999 ont la propriété d'inhiber sélectivement, *via* l'antithrombine III, le facteur Xa sans aucune activité sur la thrombine.

Il est connu que l'inhibition du facteur Xa nécessite une fixation de l'héparine sur l'AT III *via* le domaine de liaison à l'antithrombine (DLA), et que l'inhibition du facteur IIa (thrombine) nécessite une fixation à l'AT III, *via* le DLA, ainsi qu'à la thrombine *via* un domaine de liaison moins bien défini (DLT).

Les oligosaccharides de synthèse correspondant au domaine DLA de l'héparine sont connus et manifestent une activité antithrombotique dans la thrombose veineuse. Ces composés sont décrits dans EP 529715, EP 621282 et dans le brevet CA 2.040.905.

L'efficacité de ces oligosaccharides dans la prévention de la thrombose artérielle est néanmoins gênée par leur incapacité à inhiber la thrombine.

Une synthèse de glycosaminoglycanes de type héparine capables d'inhiber la thrombine *via* l'activation de l'AT III est décrite dans la demande de brevet PCT/FR 97/01344.

Dans cette demande de brevet sont décrits de nouveaux dérivés polysaccharidiques biologiquement actifs. Ils sont en particulier anticoagulants et antithrombotiques. De plus, du fait de l'obtention par synthèse de ces polysaccharides, il est possible de modifier sélectivement leur structure, et en particulier d'éliminer des substituants sulfates non désirés intervenant dans l'interaction avec certaines protéines basiques telles que le facteur plaquettaire 4 (FP4) qui est relargué lors de l'activation des plaquettes, neutralisant de façon importante l'héparine à proximité du caillot. Ainsi, on peut obtenir des polysaccharides qui sont de puissants agents antithrombotiques et anticoagulants et qui, de plus, peuvent échapper *in vivo* à l'action de protéines telles que le FP4, qui neutralisent l'effet de l'héparine en particulier sur la thrombine.

Il a en particulier été montré que ces polysaccharides sulfatés et alkylés peuvent être de puissants antithrombotiques et des anticoagulants selon la disposition des groupes alkyles et des groupes sulfates portés par le squelette glucidique.

De façon plus générale, il a été trouvé que par réalisation de séquences polysaccharidiques il est possible de moduler avec précision les activités de type GAGs pour obtenir des produits très actifs présentant les propriétés de l'héparine.

Cependant l'usage en thérapeutique humaine de certains produits décrits dans la demande de brevet PCT/FR97/01344 peut s'avérer délicate, en particulier si ces produits possèdent une longue demi-vie. Dans le domaine de la prévention ou du traitement de la thrombose avec les produits ci-dessus on doit rétablir ou maintenir la fluidité du sang tout en évitant de provoquer une hémorragie. Le caractère vital des fonctions auxquelles on s'adresse fait qu'il est préférable de ne mettre en oeuvre pour obtenir cet équilibre, que des composés possédant un courte demi-vie plus facilement manipulables.

Il est en effet bien connu que, pour une cause accidentelle quelconque, une hémorragie peut se déclencher chez un patient sous traitement. On peut également avoir besoin d'intervenir chirurgicalement chez un malade sous traitement antithrombotique. Ces différentes raisons font également que l'on préfère utiliser des composés à courte demi-vie.

La demi-vie des polysaccharides de synthèse de l'invention a été déterminée chez le rat où l'on a constaté, de façon inattendue, qu'elle est influencée par la structure de la molécule et en particulier du DLA, du DLT (domaine de liaison à la thrombine), et de l'espaceur.

Ainsi la présente invention concerne de nouveaux polysaccharides de synthèse, de structure proche de celle des composés de la demande de brevet PCT/FR97/01344 mais possédant des propriétés particulières. En effet, ces composés sont d'une façon inattendue pourvus d'une demi-vie d'élimination, évaluée après administration intraveineuse chez le rat, au moyen de l'activité anti-Xa, inférieure à 1 heure et demi.

Ces composés sont des hexadécasaccharides comprenant trois séquences distinctes : une séquence pentasaccharidique sulfatée DEFGH, une séquence heptasaccharidique non sulfatée Z(MN)₃ et une séquence tétrasaccharidique sulfatée VWXY.

Les composés selon la présente invention sont des polysaccharides de synthèse sous forme acide et leurs sels pharmaceutiquement acceptables avec des cations pharmaceutiquement acceptables, dont la forme anionique répond à l'une des formules (I), (II), (III), (IV) ou (V) suivantes :

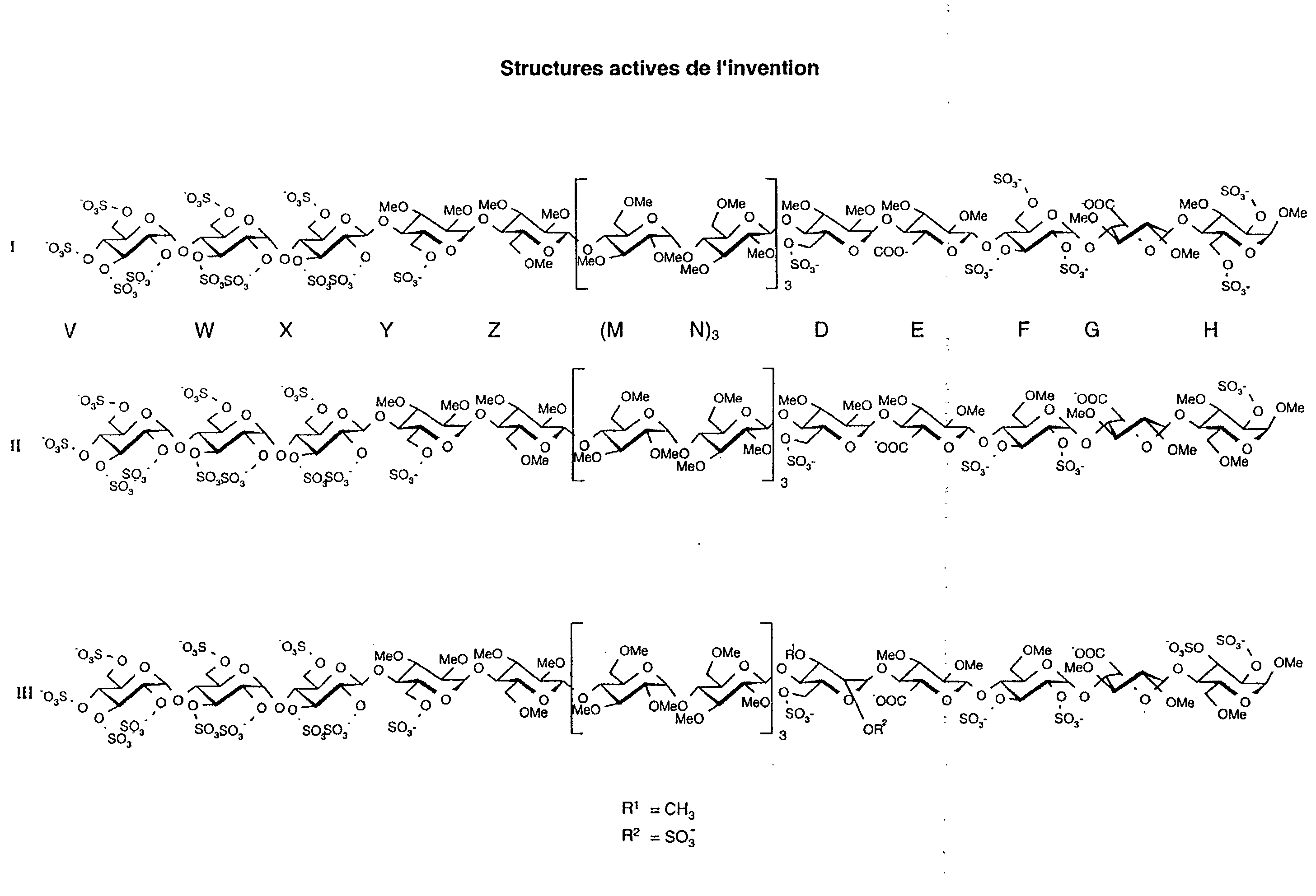

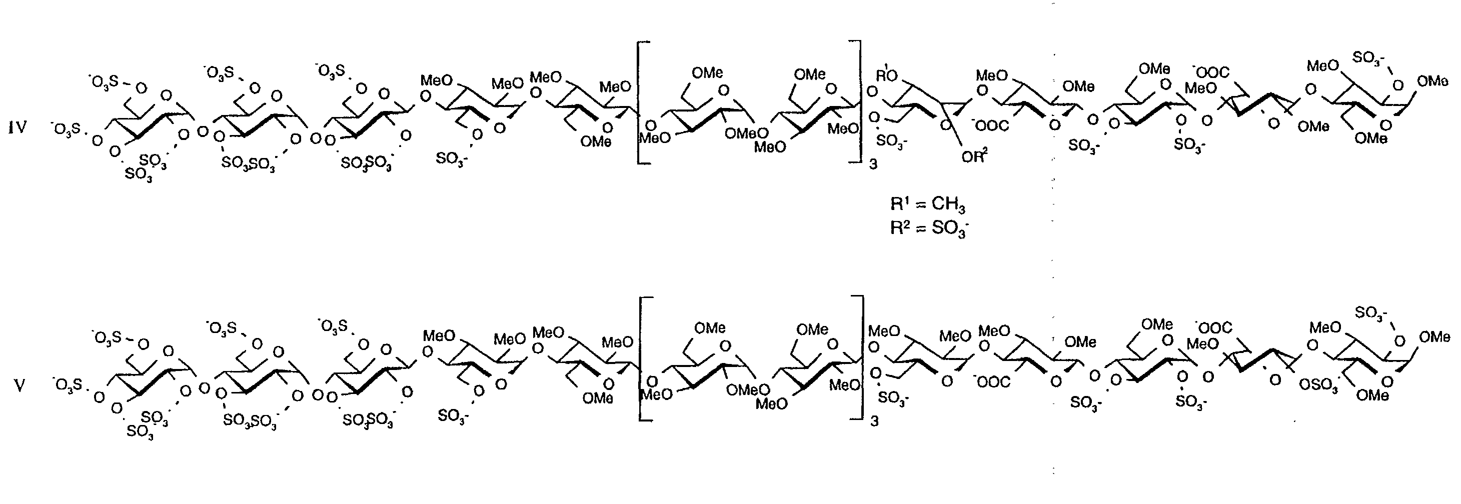

L'invention englobe les polysaccharides sous leur forme acide ou sous la forme de l'un quelconque de leurs sels pharmaceutiquement acceptables. Dans la forme acide, les fonctions -COO⁻ et -SO₃⁻ sont respectivement sous forme -COOH et -SO₃H.

On entend par sel pharmaceutiquement acceptable des polysaccharides de l'invention, un polysaccharide dans lequel une ou plusieurs des fonctions -COO⁻ ou/et -SO₃⁻ sont liées de façon ionique à un cation métallique pharmaceutiquement acceptable. Les sels préférés selon l'invention sont ceux dont le cation est choisi parmi les cations des métaux alcalins et plus préférablement encore ceux dont le cation est Na⁺ ou K⁺.

La présente invention concerne également un procédé pour la préparation des polysaccharides de l'invention.

Dans son principe ce procédé utilise des synthons di- ou oligosaccharidiques préparés comme précédemment rapporté dans la littérature. On se réfèrera notamment à EP 300099, EP 529715, EP 621282 et EP 649854 ainsi qu'à C. van Boeckel, M Petitou, Angew. Chem. Int. Ed. Engl., 1993, 32, 1671-1690. Ces synthons sont ensuite couplés les uns aux autres de façon à fournir un précurseur entièrement protégé d'un hexadécasaccharide selon l'invention, lequel est ensuite sélectivement déprotégé et sulfaté pour livrer l'hexadécasaccharide de l'invention.

Dans les réactions de couplage évoquées ci-dessus, un di- ou oligosaccharide "donneur", activé sur son carbone anomère, réagit avec un di- ou oligosaccharide "accepteur", possédant un hydroxyle libre.

La synthèse des hexadécasaccharides de l'invention peut être réalisée en suivant la séquence d'opérations suivantes, lesquelles font référence aux unités saccharidiques telles que représentées à la formule (I) ci-dessus.

On prépare d'abord un précurseur protégé de GH, pGH, possédant une fonction alcool en position 4' et un précurseur protégé de EF, pEF, activé sur son carbone anomère. Les intermédiaires pGH et pEF réagissent l'un sur l'autre pour donner EFGH. La position 4 de l'unité terminale non-réductrice est alors déprotégée pour donner un précurseur pEFGH.

En parallèle on prépare selon la même stratégie un précurseur de la partie YZ(MN)₃D, activé sur son carbone anomère, pYZ(MN)₃D.

Le tétrasaccharide pEFGH réagit ensuite sur pYZ(MN)₃D pour donner YZ(MN)₃DEFGH. L'unité terminale non-réductrice est déprotégée pour donner un précurseur pYZ(MN)₃DEFGH.

On prépare en parallèle un précurseur de VWX, pVWX.

La réaction entre pVWX et pYZ(MN)₃DEFGH livre alors le précurseur direct de l'hexadécasaccharide qui est déprotégé et sulfaté pour donner l'hexadécasaccharide attendu.

Il est évident pour l'homme du métier que plusieurs stratégies de synthèse sont possibles selon les accepteurs et donneurs que l'on choisit. On peut, par exemple, préparer un donneur VWXYZ(MN)₃D et le coupler sur EFGH pour obtenir un précurseur de l'hexadécasaccharide souhaité. On pourra se référer par exemple aux références ci-dessus et à Monosaccharides, Their chemistry and their roles in natural products, P.M. Collins et R.J. Ferrier, J; Wiley & sons, 1995 et à G.J. Boons, Tetrahedron, 1996, 52, 1095-1121.

Les composés selon l'invention ont fait l'objet d'études biochimiques et pharmacologiques qui ont montré qu'ils possèdent par ailleurs les propriétés très intéressantes des produits décrits dans la demande de brevet PCT/FR97/01344

Les composés de la présente invention qui se lient sélectivement à l'AT III avec une affinité égale ou supérieure à celle de l'héparine, possèdent des propriétés anticoagulantes et antithrombotiques supérieures à celles de l'héparine.

L'activité antithrombotique globale des produits de formule (I) à (V) a été évaluée par voie intraveineuse ou sous cutanée chez le rat, dans un modèle de stase veineuse et induction par la thromboplastine, selon la méthode décrite par J. Reyers *et al*. dans Thrombosis Research, 1980, *18*, 669-674 ainsi que dans un modèle de thrombose artérielle constitué d'un shunt implanté entre l'artère carotide et la veine jugulaire de rats tel que décrit par Umetsu *et al*. Thromb. Haemost., 1978, *39,* 74-83. Dans ces deux modèles expérimentaux, la DE₅₀ des composés de l'invention est au moins du même ordre ou inférieure à celle des autres héparinoïdes de synthèse déjà connus (DE₅₀ comprises entre 1 et 50 nmol/kg). Les composés de l'invention présentent donc une spécificité d'action et une activité anticoagulante et antithrombotique particulièrement intéressantes.

Grâce à leur activité biochimique et pharmaceutique, les composés de la présente invention constituent des médicaments très intéressants. Leur toxicité est parfaitement compatible avec cette utilisation. Ils sont également très stables et sont donc particulièrement appropriés pour constituer le principe actif de spécialités pharmaceutiques.

De plus, les composés de l'invention ne sont pas neutralisés par de fortes doses de protéines cationiques plaquettaires telles que le FP4 relarguées lors de l'activation des plaquettes durant le processus de thrombose. Les composés de l'invention sont donc tout particulièrement intéressants pour le traitement et la prévention des thromboses d'origine à la fois artérielle et veineuse.

Ils peuvent être utilisés dans diverses pathologies consécutives à une modification de l'homéostasie du système de la coagulation apparaissant en particulier lors des troubles du système cardio-vasculaire et cérébro-vasculaire comme les troubles thromboemboliques associés à l'arthérosclérose et au diabète tels l'angine instable, l'attaque cérébrale, la resténose après angioplastie, l'endartérectomie, la pose de prothèses endovasculaires ; ou les troubles thromboemboliques associés à la rethrombose après thrombolyse, à l'infarctus, à la démence d'origine ischémique, aux maladies artérielles périphériques, à l'hémodialyse, aux fibrillations auriculaires ou encore lors de l'utilisation de prothèses vasculaires de pontages aorto-coronariens. Ce produit peut par ailleurs être utilisé pour le traitement ou la prévention de pathologies thromboemboliques d'origine veineuse telles les embolies pulmonaires. Il peut être utilisé ou pour prévenir ou pour traiter les complications thrombotiques apparaissant lors d'interventions chirurgicales ou conjointement à d'autres pathologies telles que cancer, infections bactériennes ou virales. Dans le cas de son utilisation lors de la pose de prothèses, le composé de la présente invention peut recouvrir des prothèses et les rendre ainsi hémocompatibles. En particulier, ils peuvent être fixés à des prothèses intravasculaires (stents). Dans ce cas, ils peuvent éventuellement être modifiés chimiquement par introduction à l'extrémité non réductrice ou réductrice d'un bras approprié, comme décrit selon EP 649 854.

Les composé de la présente invention peuvent également être utilisés comme adjuvant lors d'endartérectomie réalisée avec des ballonnets poreux.

Les composés de l'invention sont très stables et sont donc ainsi particulièrement appropriés pour constituer le principe actif de médicaments.

Ainsi les composés selon l'invention peuvent être utilisés pour la préparation de médicaments destinés à traiter les maladies ci-dessus.

Selon un autre de ses aspects, la présente invention a donc pour objet une composition pharmaceutique contenant, en tant que principe actif, un polysaccharide de synthèse tel que défini ci-dessus.

L'invention concerne de préférence, des compositions pharmaceutiques contenant comme principe actif, un composé selon l'invention sous forme acide ou l'un de ses sels pharmaceutiquement acceptables, éventuellement en association avec un ou plusieurs excipients inertes et appropriés. De façon générale, les polysaccharides de l'invention sont utilisables dans le traitement des pathologies dépendantes d'un dysfonctionnement de la coagulation.

Dans chaque unité de dosage le principe actif est présent dans les quantités adaptées aux doses journalières envisagées. En général, chaque unité de dosage est convenablement ajustée selon le dosage et le type d'administration prévu, par exemple comprimés, gélules et similaires, sachets, ampoules, sirops et similaires, gouttes, patch transdermique ou transmuqueux de façon à ce qu'une telle unité de dosage contienne de 0,1 à 100 mg de principe actif, de préférence 0,5 à 50 mg.

Les composés selon l'invention peuvent également être utilisés en association avec un ou plusieurs autres principes actifs utiles pour la thérapeutique souhaitée tels que par exemple des antithrombotiques, des anticoagulants, des antiagrégants plaquettaires tels que par exemple le dipyridamole, l'aspirine, la ticlopidine, le clopidogrel ou des antagonistes du complexe de la glycoproteine IIb/IIIa.

Les compositions pharmaceutiques sont formulées pour l'administration aux mammifères, y compris l'homme, pour le traitement des maladies susdites.

Les compositions pharmaceutiques ainsi obtenues sont présentées avantageusement sous des formes diverses, telles que par exemple, dès solutions injectables ou buvables, dragées, comprimés ou gélules. Les solutions injectables sont les formes pharmaceutiques préférées. Les compositions pharmaceutiques de la présente invention sont notamment utiles pour le traitement à titre préventif ou curatif, des troubles de la paroi vasculaire, tels que l'athérosclérose, les états d'hypercoagulabilité observés par exemple à la suite d'opérations chirurgicales de développements tumoraux ou de dérèglements de la coagulation, induits par des activateurs bactériens, viraux, ou enzymatiques. La posologie peut largement varier en fonction de l'âge, du poids et de l'état de santé du patient, de la nature et de la sévérité de l'affection, ainsi que de la voie d'administration. Cette posologie comprend l'administration d'une ou plusieurs doses d'environ 0,1 mg à 100 mg par jour, de préférence d'environ 0,5 à 50 mg par jour, par voie intramusculaire ou sous cutanée, en administrations continues ou à intervalles réguliers.

La présente invention a donc également pour objet les compositions pharmaceutiques qui contiennent à titre de principe actif un des composés ci-dessus éventuellement en association avec un autre principe actif. Ces compositions sont réalisées de façon à pouvoir être administrées par la voie digestive ou parentérale.

Dans les compositions pharmaceutiques de la présente invention pour l'administration orale, sublinguale, sous-cutanée, intramusculaire, intraveineuse, transdermique, transmuqueux, locale ou rectale, l'ingrédient actif peut être administré sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Pour une administration transmuqueuse le principe actif peut être formulé en présence d'un promoteur tel qu'un sel biliaire, d'un polymère hydrophile tel que par exemple l'hydroxypropylcellulose, l'hydroxypropylméthylcellulose, l'hydroxyéthylcellulose, l'éthylcellulose, la carboxyméthylcellulose, le dextran, la polyvinylpyrrolidone, les pectines, les amidons, la gélatine, la caséine, les acides acryliques, les esters acryliques et leurs copolymères, les polymères ou copolymères de vinyle, les alcools vinyliques, les alcoxypolymères, les polymères d'oxyde de polyéthylène, les polyéthers ou leur mélange.

Le principe actif peut être formulé également sous forme de microcapsules, éventuellement avec un ou plusieurs supports ou additifs.

Le principe actif peut être également présenté sous forme de complexe avec une cyclodextrine, par exemple α, β ou γ cyclodextrine, 2-hydroxypropyl-β-cyclodextrine ou méthyl-β-cyclodextrine.

Le principe actif peut également être libéré par un ballonnet le contenant ou par un extenseur endovasculaire introduit dans les vaisseaux sanguins. L'efficacité pharmacologique du principe actif n'est ainsi pas affectée.

L'administration par voie sous-cutanée est la voie préférée.

Les méthodes, les préparations et les schémas suivants illustrent la synthèse des différents intermédiaires utiles à l'obtention des polysaccharides selon l'invention.

Les abréviations suivantes sont utilisées :

TBDMS : *tert*-butyldiméthylsilyle ; Lev : lévulinoyle ; Bn : benzyle ; Bz : benzoyle ; CCM : chromatographie sur couche mince ; Olm : trichloroacétimidoyle ; LSIMS : est le sigle anglais de *Liquid Secondary Ion Mass Spectrometry ;* ESIMS : est le sigle anglais de *Electron Spray Ionisation Mass Spectrometry;* TMS : triméthylsilyle ; TSP : triméthylsilyle tétradeuterio propionate de sodium ; Tf: triflate ; MS : tamis moléculaire; PMB: *p*-méthoxybenzyle ; MP : *p*-méthoxyphényle ; TS : tosyle ; ET : éthyle ; Ph : phényle ; Me : méthyle ; Ac : acétyle.

Dowex® , Sephadex® , Chelex® , Toyopearl® sont des marques déposées.

Dans les méthodes, les préparations et dans les exemples décrits ci-après, des modes opératoires généraux concernant le couplage catalytique des imidates, le clivage des esters lévuliniques, le couplage catalytique des thioglycosides, la saponification, la méthylation et la déprotection sélective du groupe p-méthoxybenzyle, la déprotection et la sulfatation des oligo- et des polysaccharides par hydrogénolyse des esters ou des éthers benzyliques, la saponification des esters ou encore les sulfatations peuvent être réalisés en appliquant les méthodes générales ci-après aux intermédiaires appropriés.

Dans la suite, des exemples de synthèse des composés de l'invention sont détaillés à titre d'illustration.

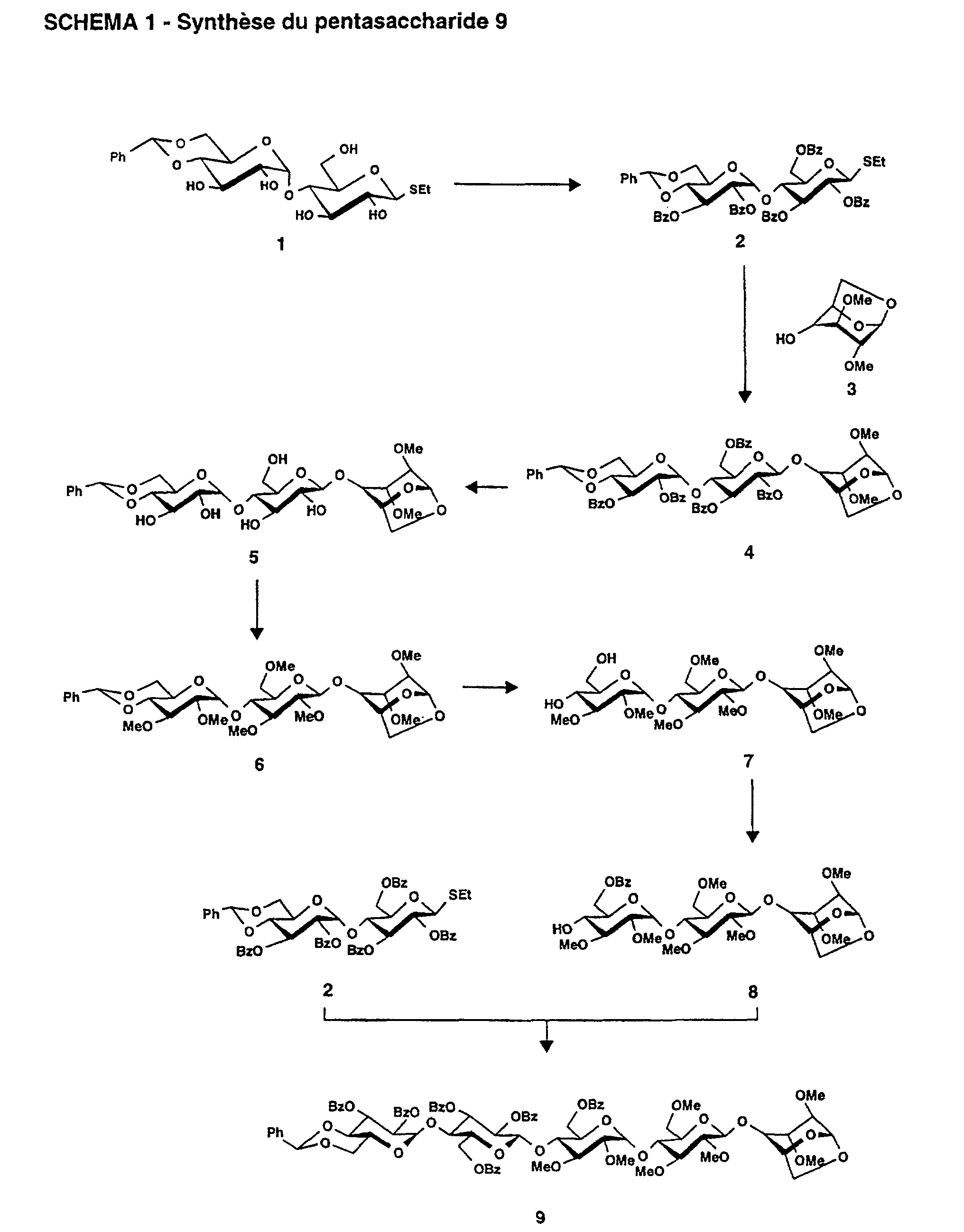

### PRÉPARATION 1

### Éthyl O-(2,3-di-O-benzoyl-4,6-O-benzylidène-α-D-glucopyranosyl)-(1→4)-2,3,6-tri-Obenzoyl-1-thio-β-D-glucopyranoside (2)

A une solution refroidie (0° C) du composé 1 (16,7 g, 35,2 mmol) (J. Westman and M. Nilsson, J. Carbohydr. Chem., 1995, 14 (7), 949-960) dans la pyridine (202 ml), on ajoute pendant 20 minutes goutte à goutte du chlorure de benzoyle (24,5 ml, 211 mmol). On agite pendant 20 heures le mélange réactionnel à température ambiante ; la CCM révèle une conversion de 50 % environ. Le mélange est dilué avec de l'eau et du dichlorométhane. Après extraction, la phase organique est lavée avec une solution d'hydrogénocarbonate de sodium à 10 %, de l'eau, séchée sur sulfate de magnésium et concentrée. Le résidu est à nouveau traité avec du chlorure de benzoyle selon le mode opératoire décrit ci-dessus. Le produit brut est purifié par une chromatographie sur colonne de gel de silice pour fournir 22 g du composé 2.

CCM : Rf = 0,80, gel de silice, toluène/éthanol 9/1 v/v

### PRÉPARATION 2

### O-(2,3-Di-O-benzoyl-4,6-O-benzylidène-α-D-glucopyranosyl)-(1→4)-O-(2,3,6-tri-Obenzoyl-β-D-glucopyranosyl)-(1→4)-1,6-anhydro-2,3-di-O-méthyl-β-D-glucopyranose (4)

Un mélange de thioglycoside 2 (1,05 g, 1,05 mmol), du composé 3 (200 mg, 1,05 mmol) (Jeanloz *et al*., J. Org. Chem. 1961, 26, 3939-3944) et d'un tamis moléculaire 4Å en poudre (1,1 g) dans le toluène (18 ml) est agité sous atmosphère d'azote pendant 15 minutes. Le mélange est ensuite refroidi à - 20° C et une solution fraîchement préparée de *N*-iodosuccinimide (1,11 mmol) et d'acide trifluorométhanesulfonique (0,125 mmol) dans le dichlorométhane/dioxane 1/1 v/v (6 ml) y est introduite. Après 10 minutes, le mélange réactionnel rouge est filtré, dilué avec du dichlorométhane, extrait, successivement lavé avec une solution de thiosulfate de sodium à 10 %, une solution d'hydrogénocarbonate de sodium à 10% et de l'eau, séché sur sulfate de magnésium puis concentré sous vide. La purification du résidu est effectuée par chromatographie sur une colonne de gel de silice afin d'obtenir 1,25 g du composé 4.

CCM : Rf = 0,55, gel de silice, heptane/acétate d'éthyle 4/6 v/v

### PRÉPARATION 3

### O-(4,6-O-Benzylidène-α-D-glucopyranosyl)-(1→4)-O-(β-D-glucopyranosyl)-(1→4)-1,6-anhydro-2,3-di-O-méthyl-β-D-glucopyranose (5)

A une solution du composé 4 (1,24g, 1,11 mmol) dans du méthanol/dioxane 1/1 v/v (7 ml), on ajoute du *tert*-butylate de potassium (environ 50 mg). On agite pendant une heure, puis on ajoute à nouveau 50 mg de *tert*-butylate de potassium ; le mélange est ensuite agité pendant encore 60 minutes. Le mélange réactionnel est neutralisé avec une résine Dowex® 50WX8 H⁺, filtré et concentré sous vide. Après une chromatographie sur colonne de gel de silice, 665 mg du composé 5 sont isolés sous forme d'huile.

CCM : Rf = 0,50, gel de silice, dichlorométhane/méthanol 85/15 v/v

### PRÉPARATION 4

### O-(4,6-O-Benzylidène-2,3-di-O-méthyl-α-D-glucopyranosyl)-(1→4)-O-(2,3,6-tri-Ométhyl-β-D-glucopyranosyl)-(1→4)-1,6-anhydro-2,3-di-O-méthyl-β-D-glucopyranose (6)

A une solution refroidie (5°C) du composé 5 (660 mg, 1,1 mmol) dans du tétrahydrofurane sec (8 ml), on ajoute de l'hydrure de sodium (387 mg, 9,65 mmol) sous atmosphère d'azote. On ajoute goutte à goutte de l'iodure de méthyle (0,51 ml, 8,22 mmol) et le mélange est agité pendant 20 heures à température ambiante. L'excès d'hydrure de sodium est détruit avec du méthanol et le mélange est versé dans 50 ml d'eau glacée. Après extraction avec de l'acétate d'éthyle (3 fois 20 ml), la phase organique est lavée avec une solution de chlorure de sodium, séchée sur sulfate de magnésium et concentrée pour donner 690 mg du composé 6.

CCM : Rf = 0,25, gel de silice, dichlorométhane/méthanol 95/5 v/v

### PRÉPARATION 5

### O-(2,3-Di-O-méthyl-α-D-glucopyranosyl)-(1→4)-O-(2,3,6-tri-O-méthyl-β-D-glucopyranosyl)-(1→4)-1,6-anhydro-2,3-di-O-méthyl-β-D-glucopyranose (7)

Le composé 6 (690 mg, 1,03 mmol) est dissous dans de l'acide acétique à 80 % (7,3 ml) et agité pendant 20 heures à 40° C. Le mélange est concentré sous vide et co-évaporé avec du toluène. Une chromatographie sur colonne de gel de silice dans du dichlorométhane/acétate d'éthyle/méthanol 8/1/1 permet d'obtenir 569 mg du composé 7.

CCM : Rf = 0,40, gel de silice, dichlorométhane/méthanol 9/1 v/v

### PRÉPARATION 6

### O-(6-O-Benzoyl-2,3-di-O-méthyl-α-D-glucopyranosyl)-(1→4)-O-(2,3,6-tri-O-méthyl-β-D-glucopyranosyl)-(1→4)-1,6-anhydro-2,3-di-O-méthyl-β-D-glucopyranose (8)

À une solution du composé 7 (560 mg, 0,96 mmol) dans du dichlorométhane, on ajoute du 1-benzoyloxy-1*H*-benzotriazole (227 mg, 1,05 mmol) et de la triéthylamine (1,15 mmol) puis le mélange est agité pendant 20 heures à température ambiante. Le mélange réactionnel est dilué avec du dichlorométhane, lavé avec une solution d'hydrogénocarbonate de sodium à 10 % et de l'eau. La phase organique est séchée sur sulfate de magnésium, filtrée et évaporée à sec. Le produit est purifié par chromatographie sur colonne de gel de silice afin d'obtenir 600 mg du composé 8.

CCM : Rf = 0,50, gel de silice, dichlorométhane/méthanol 9/1 v/v

### PRÉPARATION 7

### O-(2,3-Di-O-benzoyl-4,6-O-benzylidène-α-D-glucopyranosyl)-(1→4)-O-(2,3,6-tri-Obenzoyl-β-D-glucopyranosyl)-(1→4)-O-(6-O-benzoyl-2,3-di-O-méthyl-α-D-glucopyranosyl)-(1→4)-O-(2,3,6-tri-O-méthyl-β-D-glucopyranosyl)-(1→4)-1,6-anhydro-2,3-di-O-méthyl-β-D-glucopyranose (9)

Le composé 8 est transformé en composé 9 selon le mode opératoire décrit pour la préparation du composé 4. La réaction de couplage est réalisée à 5° C.

CCM : Rf = 0,50, gel de silice, heptane/acétate d'éthyle 2/8 v/v.

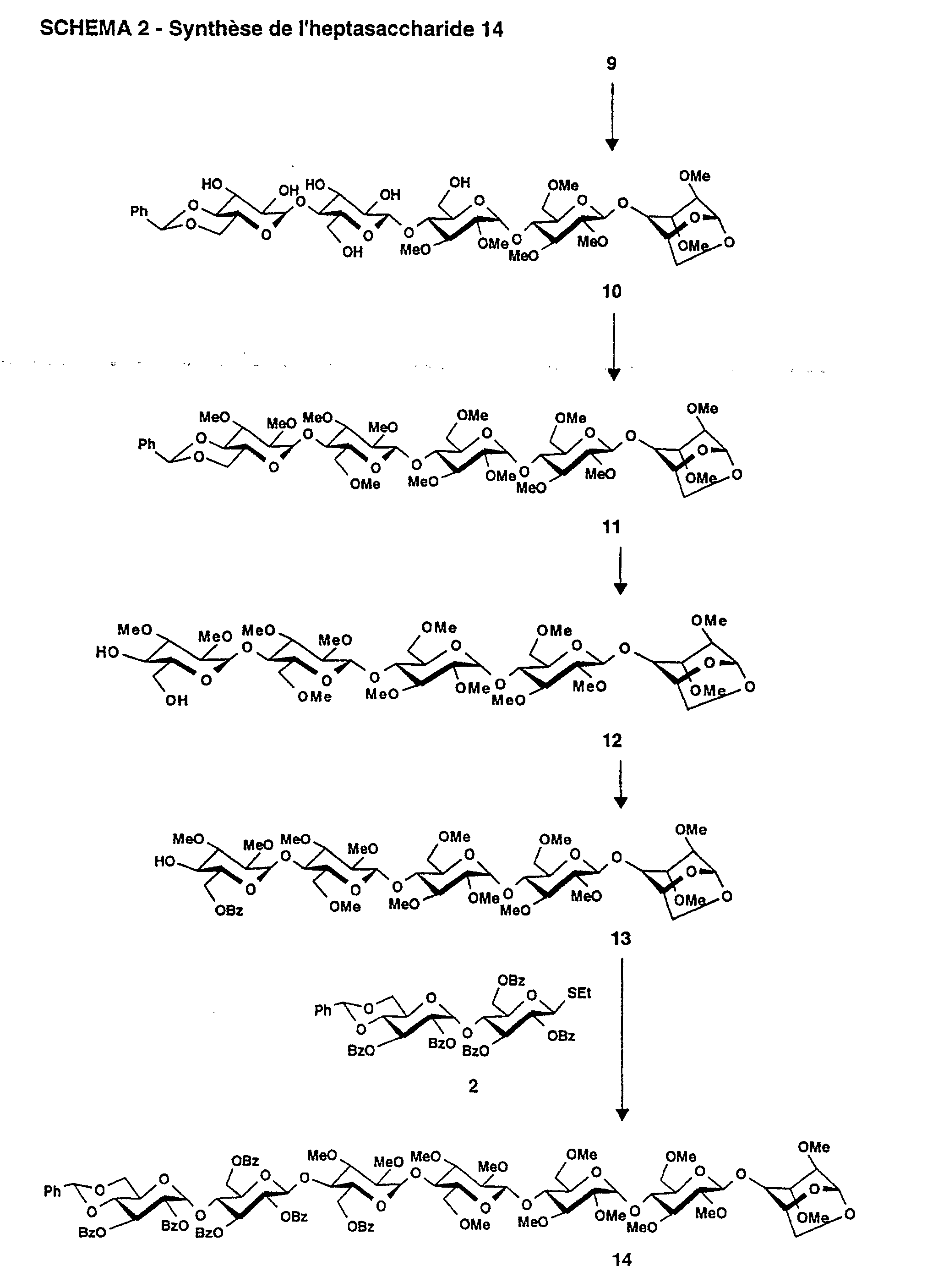

### PRÉPARATION 8

### O-(4,6-O-Benzylidène-α-D-glucopyranosyl)-(1→4)-O-(β-D-glucopyranosyl)-(1→4)-O(2,3-di-O-méthyl-α-D-glucopyranosyl)-(1→4)-O-(2,3,6-tri-O-méthyl-β-D-glucopyranosyl)-(1→4)-1,6-anhydro-2,3-di-O-méthyl-β-D-glucopyranose (10)

Le composé 9 est transformé en composé 10 selon le même mode opératoire que celui décrit pour la préparation du composé 5.

CCM : Rf = 0,35, gel de silice, dichlorométhane/méthanol/ 9/1 v/v

### PRÉPARATION 9

### O-(4,6-O-Benzylidène-2,3-di-O-méthyl-α-D-glucopyranosyl)-(1→4)-O-(2,3,6-tri-O-méthyl-β-D-glucopyranosyl)-(1→4)-O-(2,3,6-tri-O-méthyl-α-D-glucopyranosyl)-(1→4)-O-(2,3,6-tri-O-méthyl-β-D-glucopyranosyl)-(1→4)-1,6-anhydro-2,3-di-O-méthyl-β-D-glucopyranose (11)

Le composé 10 est transformé en composé 11 selon le même mode opératoire que celui décrit pour la préparation du composé 6.

CCM : Rf = 0,50, gel de silice, dichlorométhane/méthanol/ 9/1 v/v

### PRÉPARATION 10

### O-(2,3-Di-O-méthyl-α-D-glucopyranosyl)-(1→4)-O-(2,3,6-tri-O-méthyl-β-D-glucopyranosyl)-(1→4)-O-(2,3,6-tri-O-méthyl-α-D-glucopyranosyl)-(1→4)-O-(2,3,6-triO-méthyl-β-D-glucopyranosyl)-(1→4)-1,6-anhydro-2,3-di-O-méthyl-β-D-glucopyranose (12)

Le composé 11 est transformé en composé 12 selon le même mode opératoire que celui décrit pour la préparation du composé 7.

CCM : Rf = 0,35, gel de silice, dichlorométhane/méthanol/ 9/1 v/v

### PRÉPARATION 11

### O-(6-O-Benzoyl-2,3-di-O-méthyl-α-D-glucopyranosyl)-(1→4)-O-(2,3,6-tri-O-méthyl-β-D-glucopyranosyl)-(1→4)-O-(2,3,6-tri-O-méthyl-α-D-glucopyranosyl)-(1→4)-O-(2,3,6-tri-O-méthyl-β-D-glucopyranosyl)-(1→4)-1,6-anhydro-2,3-di-O-méthyl-β-D-glucopyranose (13)

Le composé 12 est transformé en composé 13 selon le même mode opératoire que celui décrit pour la préparation du composé 8.

CCM : Rf = 0,40, gel de silice, toluène/acétate d'éthyle/éthanol 7,0/1,5/1,5 v/v/v

### PRÉPARATION 12

### O-(2,3-Di-O-benzoyl-4,6-O-benzylidène-α-D-glucopyranosyl)-(1→4)-O-(2,3,6-tri-O-benzoyl-β-D-glucopyranosyl)-(1→4)-O-(6-O-benzoyl-2,3-di-O-méthyl-α-D-glucopyranosyl)-(1→4)-O-(2,3,6-tri-O-méthyl-β-D-glucopyranosyl)-(1→4)-O-(2,3,6-triO-méthyl-α-D-glucopyranosyl)-(1→4)-O-(2,3,6-tri-O-méthyl-β-D-glucopyranosyl)-(1→4)-1,6-anhydro-2,3-di-O-méthyl-β-D-glucopyranose (14)

La réaction de couplage du composé 13 avec le disaccharide 2 est réalisée selon le mode opératoire décrit pour la préparation du composé 9, pour fournir le composé 14.

CCM : Rf = 0,40, gel de silice, toluène/acétate d'éthyle/éthanol 7,0/1,5/1,5 v/v/v

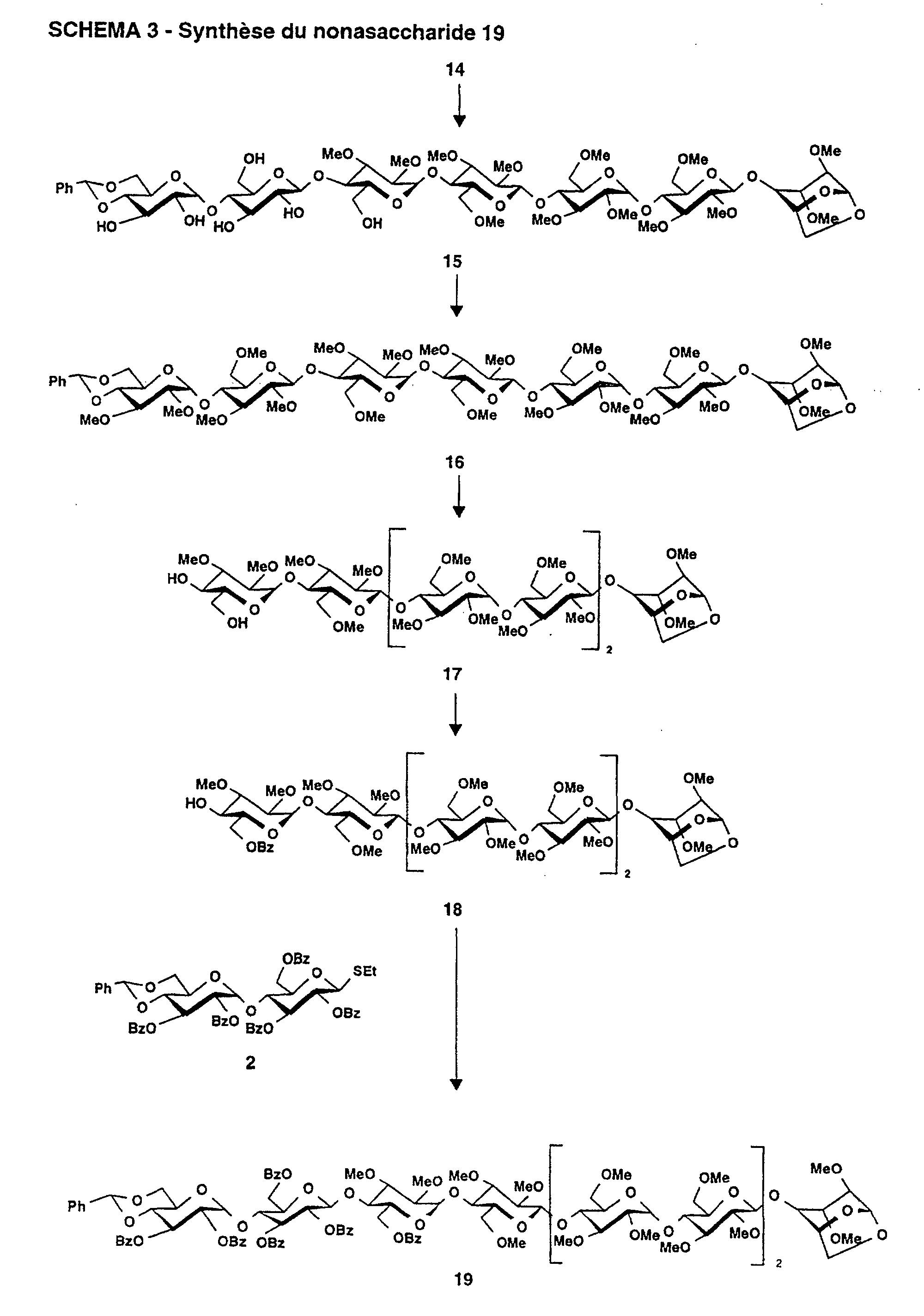

### PRÉPARATION 13

### O-(4,6-O-Benzylidène-α-D-glucopyranosyl)-(1→4)-O-(β-D-glucopyranosyl)-(1→4)-O-(2,3-di-O-méthyl-α-D-glucopyranosyl)-(1→4)-O-(2,3,6-tri-O-méthyl-β-D-glucopyranosyl)-(1→4)-O-(2,3,6-tri-O-méthyl-α-D-glucopyranosyl)-(1→4)-O-(2,3,6-tri-O-méthyl-β-D-glucopyranosyl)-(1→4)-1,6-anhydro-2,3-di-O-méthyl-β-D-glucopyranose (15)

Le composé 14 est transformé en composé 15 selon le même mode opératoire que celui décrit pour la préparation du composé 5.

CCM : Rf = 0,60, gel de silice, dichlorométhane/méthanol 9/1 v/v

### PRÉPARATION 14

### O-(4,6-O-Benzylidène-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-a-D-glucopyranosyl)-(1→4)-O-(2,3,6-tri-O-méthyl-β-D-glucopyranosyl)-(1→4)]₂-1,6-anhydro-2,3-di-O-méthyl-β-D-glucopyranose (16)

Le composé 15 est transformé en composé 16 selon le même mode opératoire que celui décrit pour la préparation du composé 6.

CCM : Rf = 0,70, gel de silice, dichlorométhane/méthanol 9/1 v/v

### PRÉPARATION 15

### O-(2,3-Di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₂-1,6-anhydro-2,3-di-O-méthyl-β-D-glucopyranose (17)

Une solution du composé 16 (5,05 g, 2,0 mmol) dans de l'acide acétique à 80 % (50 ml) est agitée pendant 20 heures à 40° C. Le mélange est concentré sous vide et co-évaporé avec du toluène. Le résidu est dissous dans de l'acétate d'éthyle et extrait avec de l'eau. La phase aqueuse est extraite avec du dichlorométhane et la phase organique est séchée sur sulfate de magnésium, filtrée et évaporée à sec pour donner 2,68 g du composé 17.

CCM : Rf = 0,50, gel de silice, dichlorométhane/méthanol 9/1 v/v

### PRÉPARATION 16

### O-(6-O-Benzoyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₂-1,6-anhydro-2,3-di-O-méthyl-β-D-glucopyranose (18)

Le composé 17 est transformé en composé 18 selon le même mode opératoire que celui décrit pour la préparation du composé 8.

CCM : Rf = 0,80, gel de silice, toluène/acétate d'éthyle/éthanol 7,0/1,5/1,5 v/v/v

### PRÉPARATION 17

### O-(2,3-Di-O-benzoyl-4,6-O-benzylidène-α-D-glucopyranosyl)-(1→4)-O-(2,3,6-tri-O-benzoyl-β-D-glucopyranosyl)-(1→4)-O-(6-O-benzoyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₂-1,6-anhydro-2,3-di-O-méthyl-β-D-glucopyranose (19)

Un mélange du thioglycoside 2 (1,97 g, 2,0 mmol, 3,5 eq), d'heptasaccharide 18 (0,86 g, 0,57 mmol) et d'un tamis moléculaire 4Å en poudre dans le toluène (22 ml) est agité sous atmosphère d'azote pendant 15 minutes. On ajoute alors goutte à goutte, à température ambiante, une solution fraîchement préparée contenant de la *N*-iodosuccinimide (496 mg, 2,2 mmol) et de l'acide trifluorométhanesulfonique (0,808 mmol) dans du dichlorométhane/dioxane 1/1 v/v (12 ml). Après 10 minutes, le mélange réactionnel est filtré, dilué avec du dichlorométhane, extrait, lavé avec une solution de thiosulfate de sodium à 10% et une solution d'hydrogénocarbonate de sodium à 10%, séché sur sulfate de magnésium et concentré sous vide. Le produit brut est purifié par chromatographie sur une colonne de gel de silice pour fournir 1,09 g du composé 19.

CCM : Rf = 0,80, gel de silice, toluène/acétate d'éthyle/éthanol 6/2/2 v/v/v.

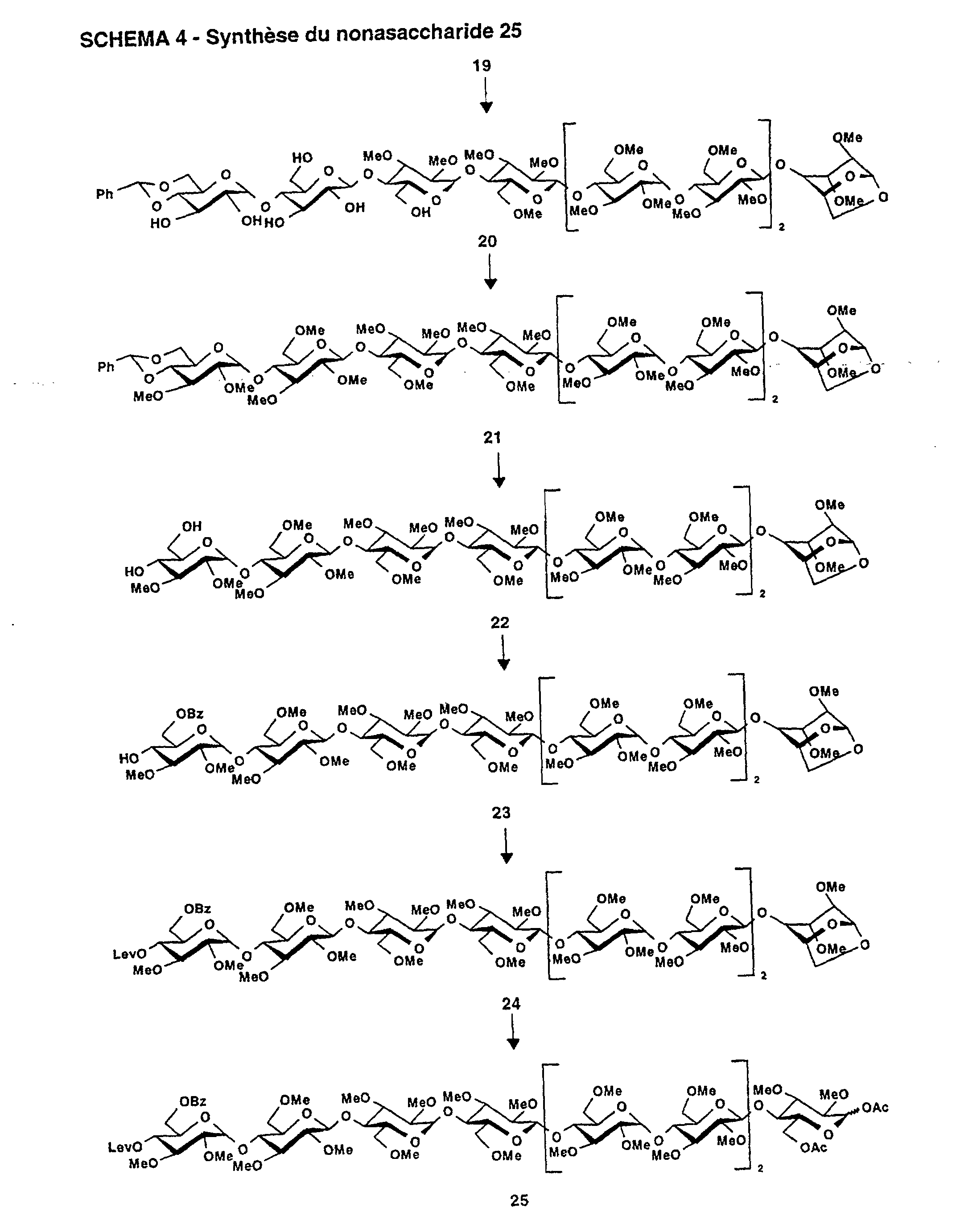

### PRÉPARATION 18

### O-(4,6-O-Benzylidène-α-D-glucopyranosyl)-(1→4)-O-(β-D-glucopyranosyl)-(1→4)-O-(2,3-di-O-méthyl-α-D-glucopyranosyl)-(1→4)-O-(2,3,6-tri-O-méthyl-β-D-glucopyranosyl)-[(1→4)-O-(2,3,6-tri-O-méthyl-α-D-glucopyranosyl)-(1→4)-O-(2,3,6-tri-O-méthyl-β-D-glucopyranosyl)]₂ - (1→4)-1,6-anhydro-2,3-di-O-méthyl-β-D-glucopyranose (20)

Le composé 19 est transformé en composé 20 selon le même mode opératoire que celui décrit pour la préparation du composé 5.

CCM : Rf = 0,25, gel de silice, toluène/acétate d'éthyleléthanol 5,0/2,5/2,5 v/v/v

### PRÉPARATION 19

### O-(4,6-O-Benzylidène-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-1,6-anhydro-2,3-di-O-méthyl-β-D-glucopyranose (21)

Le composé 20 est transformé en composé 21 selon le même mode opératoire que celui décrit pour la préparation du composé 6.

CCM : Rf = 0,50, gel de silice, toluène/acétate d'éthyle/éthanol 6/2/2 v/v/v

### PRÉPARATION 20

### O-(2,3-Di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-1,6-anhydro-2,3-di-O-méthyl-β-D-glucopyranose (22)

Le composé 21 est transformé en composé 22 selon le même mode opératoire que celui décrit pour la préparation du composé 7.

CCM : Rf = 0,20, gel de silice, toluène/acétate d'éthyle/éthanol 6/2/2 v/v/v

### PRÉPARATION 21

### O-(6-O-Benzoyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-1,6-anhydro-2,3-di-O-méthyl-β-D-glucopyranose (23)

Le composé 22 est transformé en composé 23 selon le même mode opératoire que celui décrit pour la préparation du composé 8.

CCM : Rf = 0,20, gel de silice, toluène/acétate d'éthyle/éthanol 6/2/2 v/v/v

### PRÉPARATION 22

### O-(6-O-Benzoyl-4-O-lévulinoyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-1,6-anhydro-2,3-di-O-méthyl-β-D-glucopyranose (24)

À une solution du composé 23 (320 mg, 0,167 mmol) dans du dioxane (1 ml), on ajoute du chlorhydrate de 1-(3-diméthylaminopropyl)-3-éthylcarbodiimide (48 mg, 0,25 mmol), de l'acide lévulinique (29 mg, 0,25 mmol) et de la diméthylaminopyridine (4 mg, 0,033 mmol). Le mélange réactionnel est agité pendant 3 heures à température ambiante sous atmosphère d'azote. On ajoute alors du dichlorométhane et de l'eau et après extraction, la phase organique est lavée à l'eau, séchée sur sulfate de magnésium, filtrée et concentrée. Le produit brut est purifié par chromatographie sur une colonne de gel de silice pour donner 312 mg du composé 24.

CCM : Rf = 0,50, gel de silice, toluène/acétate d'éthyle/éthanol 6/2/2 v/v/v

### PRÉPARATION 23

### O-(6-O-Benzoyl-4-O-lévulinoyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-a-D-glucopyranosyl)-(1→4)-O-(2,3,6-tri-O-méthyl-β-D-glucopyranosyl)-(1→4)]₃-1,6-di-O-acétyl-2,3-di-O-méthyl-α,β-D-glucopyranose (25)

Une solution du composé 24 (312 mg, 0,155 mmol) dans un mélange d'anhydride acétique (2,25 ml), d'acide acétique (50 µl) et d'acide trifluoroacétique (0,14 ml) est agitée pendant 4 heures à température ambiante. Après l'ajout de toluène (10 ml), le mélange est concentré et co-évaporé avec du toluène (3 fois 10 ml). Après chromatographie sur une colonne de gel de silice, 324 mg du composé 25 sont isolés.

CCM : Rf = 0,65, gel de silice, toluène/acétate d'éthyle/éthanol 6/2/2 v/v/v

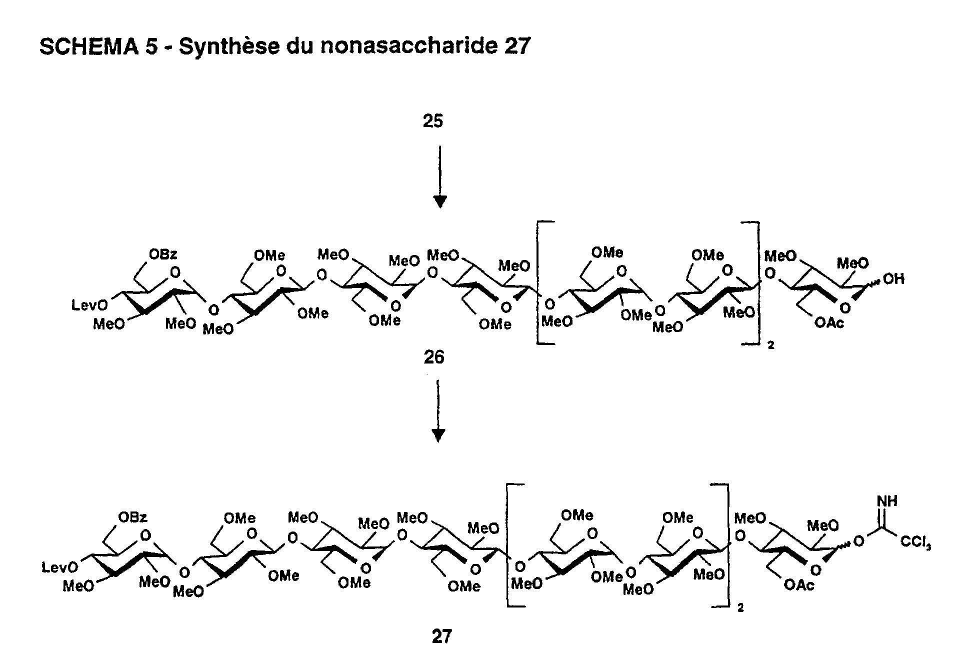

### PRÉPARATION 24

### O-(6-O-Benzoyl-4-O-lévulinoyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-6-O-acétyl-2,3-di-O-méthyl-α,β-D-glucopyranose (26)

Une solution du composé 25 (324 mg, 0,153 mmol) et de morpholine (22,3 µl, 0,256 mmol) dans du toluène (2 ml) est agitée pendant 4 heures à 35° C. Ensuite, on ajoute à nouveau de la morpholine (22,3 µl) et le mélange réactionnel est agité pendant 20 heures à 35° C. Le mélange est refroidi rapidement avec de l'eau. Après extraction avec du dichforométhane, la phase organique est successivement lavée avec de l'acide chlorhydrique 0,1 N et de l'eau, séchée et évaporée jusqu'à sec. Après chromatographie sur une colonne de gel de silice, 280 mg du composé 26 sont isolés.

CCM : Rf = 0,45, gel de silice, toluène/acétate d'éthyle/éthanol 6/2/2 v/v/v

### PRÉPARATION 25

### O-(6-O-Benzoyl-4-O-lévulinoyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-6-O-acétyl-2,3-di-O-méthyl-α,β-D-glucopyranose trichloroacétimidate (27)

Du trichloroacétonitrile (39 µl, 0,39 mmol) et du carbonate de césium (4,7 mg) sont ajoutés à une solution du composé 26 (138 mg, 0,066 mmol) dans du dichlorométhane (1,5 ml). Après agitation pendant 2 heures, le mélange est filtré, concentré et le résidu est soumis à une chromatographie sur une colonne de gel de silice pour donner 152 mg de l'imidate 27.

CCM : Rf = 0,35, gel de silice, toluène/acétate d'éthyle/éthanol 8/1/1 v/v/v

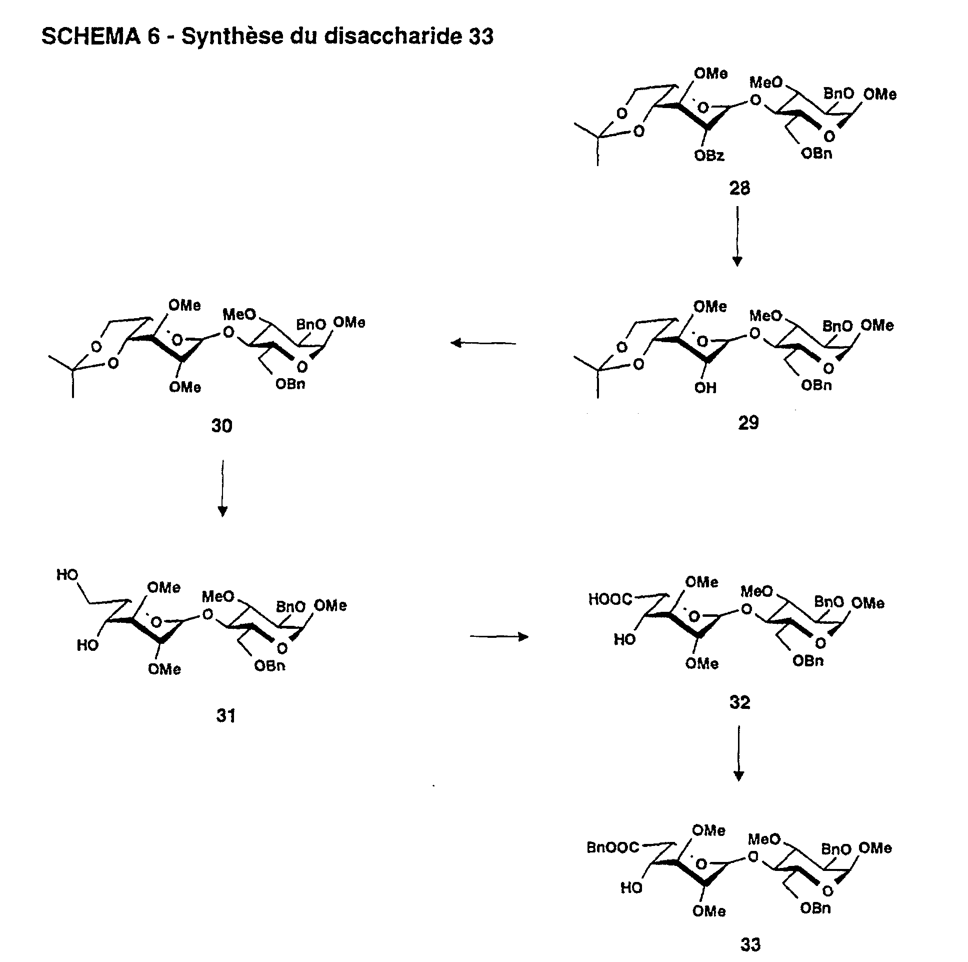

### PRÉPARATION 26

### Méthyl O-(4,6-O-isopropylidène-3-O-méthyl-α-L-idopyranosyl)-(1→4)-2,6-di-O-benzyl-3-O-méthyl-α-D-glucopyranoside (29)

Le composé 28 (2,5 g, 3,53 mmol) (M. Petitou *et al*., J. Med. Chem., 1997, *40,* 1600) est traité comme pour la synthèse de 5 et, après chromatographie sur silice (cyclohexane/acétate d'éthyle 2/1), conduit à 29 (2,1 g, 98 %).

CCM : Rf = 0,32, gel de silice, cyclohexane/acétate d'éthyle 2/1 v/v

### PRÉPARATION 27

### Méthyl O-(4,6-O-isopropylidène-2,3-di-O-méthyl-a-L-idopyranosyl)-(1→4)-2,6-di-O-benzyl-3-O-méthyl-α-D-glucopyranoside (30)

Le composé 29 (2,0 g, 3,3 mmol) est traité comme pour la synthèse de 6 et, après chromatographie sur silice (cyclohexane/acétate d'éthyle 5/1), conduit à 30 (1,83 g, 89 %).

CCM : Rf = 0,38, gel de silice, cyclohexane/acétate d'éthyle 5/2 v/v

### PRÉPARATION 28

### Méthyl O-(2,3-di-O-méthyl-α-L-idopyranosyl)-(1→4)-2,6-di-O-benzyl-3-O-méthyl-α-D-glucopyranoside (31)

A une solution du composé 30 (1,76 g, 2,84 mmol) dans du dichlorométhane (16 ml) est ajoutée une solution aqueuse d'acide trifluoroacétique à 70% (3,14 ml). Après 50 minutes, le milieu réactionnel est dilué dans du dichlorométhane puis est lavé à l'eau jusqu'à neutralisation. La phase organique est ensuite séchée (sulfate de sodium), filtrée, puis concentrée. Le résidu est purifié sur silice pour donner 31 (1,45 g, 88 %).
[α]²⁰_{D} = +10 (c = 1,0, dichlorométhane).

### PRÉPARATION 29

### Méthyl O-(acide 2,3-di-O-méthyl-α-L-idopyranosyluronique)-(1→4)-2,6-di-O-benzyl-3-O-méthyl-α-D-glucopyranoside (32)

A une solution du composé 31 (1,16 g) dans du dichlomométhane (6 ml), on ajoute du 2,2,6,6-tétraméthyl-1-pipéridinyloxy (3,3 mg), une solution d'hydrogénocarbonate de sodium (4 ml), du bromure de potassium (22 mg) et du chlorure de tétrabutylammonium (29 mg). Le mélange est refroidi à 0° C et on ajoute pendant 15 minutes un mélange d'une solution saturée de chlorure de sodium (4,4 ml), d'une solution saturée d'hydrogénocarbonate de sodium (2,2 ml) et d'hypochlorite de sodium (1,3 M, 5 ml). Après agitation pendant 1 heure, le mélange est dilué avec de l'eau et extrait (3 fois), avec du dichlorométhane. La phase organique est lavée avec une solution aqueuse de chlorure de sodium, séchée sur sulfate de magnésium, filtrée et évaporée à sec pour donner 1,34 g du composé brut 32.

Rf = 0,22, gel de silice, dichlorométhane/méthanol 10/1 v/v.

### PRÉPARATION 30

### Méthyl O-(benzyl 2,3-di-O-méthyl-α-L-idopyranosyluronate)-(1→4)-2,6-di-O-benzyl-3-O-méthyl-α-D-glucopyranoside (33)

Sous atmosphère d'azote, le composé 32 est dissous dans du N,N-diméthylformamide (11 ml). On ajoute de l'hydrogénocarbonate de potassium (0,67 g) et du bromure de benzyle (1,07 ml) et le mélange est agité pendant 90 minutes. On ajoute de l'acétate d'éthyle et de l'eau puis après extraction, la phase organique est concentrée. Une purification par chromatographie sur colonne de gel de silice permet d'obtenir 0,99 g du composé 33.

CCM : Rf = 0,58, gel de silice, toluène/éther diéthylique 1/4 v/v

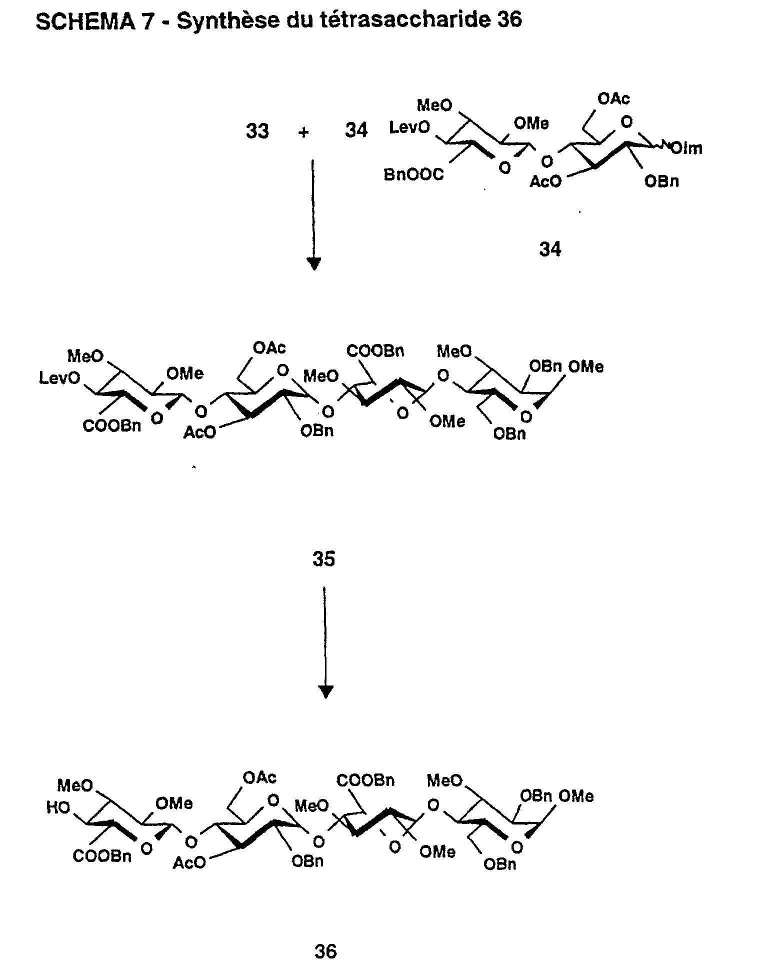

### PRÉPARATION 31

### Méthyl O-(benzyl 4-O-lévulinoyl-2,3-di-O-méthyl-β-D-glucopyranosyluronate)-(1→4)-O-(3,6-di-O-acétyl-2-O-benzyl-α-D-glucopyranosyl)-(1→4)-O-(benzyl 2,3-di-O-méthyl-α-L-idopyranosyluronate)-(1→4)-2,6-di-O-benzyl-3-O-méthyl-α-D-glucopyranoside (35)

Le composé 34 (274 mg, 0,31 mmol) (M. Petitou, *et al*. J. Med. Chem., 1997, 40, 1600) et le composé 33 (200 mg, 0,29 mmol) dans du toluène (10 ml) sont traités en présence d'un tamis moléculaire (220 mg), et à - 20°C, par du trifluorométhanesulfonate de *tert*-butyldiméthylsilyle (0,15 ml d'une solution 1 molaire dans le toluène). Après 10 minutes d'agitation, le mélange réactionnel est neutralisé (NaHCO₃), filtré et concentré. Une chromatographie sur colonne de gel de silice donne le composé 35 (334 mg, 80%).
[α]²⁰_{D} = +31 (c = 0,76, dichlorométhane).

### PRÉPARATION 32

### Méthyl O-(benzyl 2,3-di-O-méthyl-β-D-glucopyranosyluronate)-(1→4)-O-(3,6-di-O-acétyl-2-O-benzyl-α-D-glucopyranosyl)-(1→4)-O-(benzyl 2,3-di-O-méthyl-α-L-idopyranosyl uronate)-(1→4)-2,6-di-O-benzyl-3-O-méthyl-α-D-glucopyranoside (36)

Le composé 35 (308 mg, 0,22 mmol) est dissous dans un mélange toluène/éthanol (1/2, 43 ml), puis de l'acétate d'hydrazine est ajoutée (101 mg, 5eq). Après 45 minutes d'agitation, le mélange est concentré puis purifié sur colonne de silice pour donner le composé 36 (162 mg, 89 %).
[α]²⁰_{D} = +29 (c = 1,05, dichlorométhane).

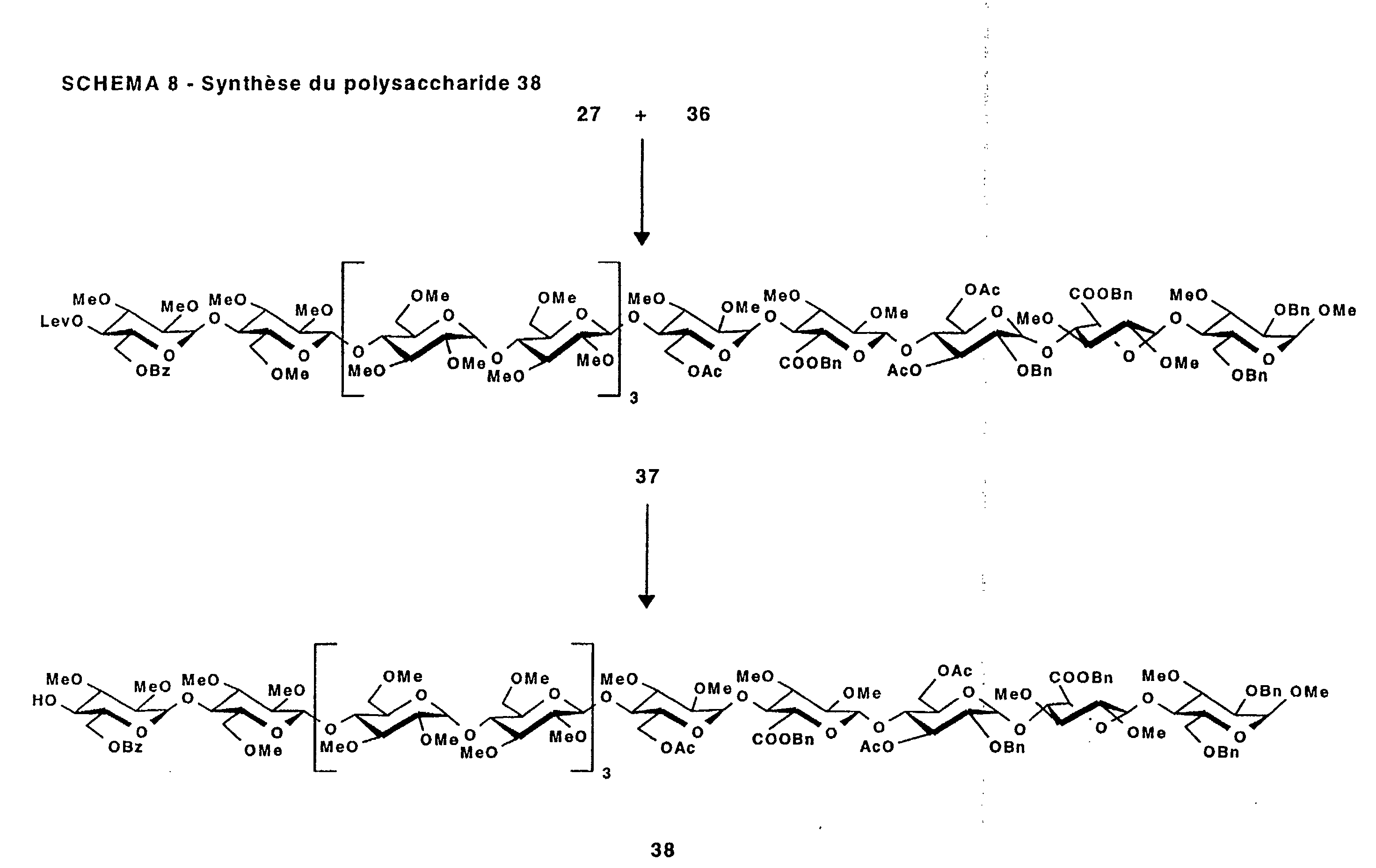

### PRÉPARATION 33

### Méthyl O-(6-O-benzoyl-4-O-lévulinoyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-gluco pyranosyl)-(1→4)-O-(2,3,6-tri-O-méthyl-β-D-glucopyranosyl)-(1→4)-]₃-O-(6-O-acétyl-2,3-di-O-méthyl-α-D-glucopyranosyl)-(1→4)-O-(benzyl 2,3-di-O-méthyl-β-D-gluco pyranosyluronate)-(1→4)-O-(3,6-di-O-acétyl-2-O-benzyl-α-D-glucopyranosyl)-(1→4)-O-(benzyl 2,3-di-O-méthyl-α-L-idopyranosyluronate)-(1→4)-2,6-di-O-benzyl-3-O-méthyl-α-D-glucopyranoside (37)

L'imidate 27 (177 mg, 76,6 µmol) et l'accepteur 36 (201 mg, 0,15 mmol) dans un mélange éther diéthylique/dichlorométhane 2/1 (2,8 ml) sont traités par du trifluorométhanesulfonate de *tert*-butyldiméthylsilyle (11,5 µl d'une solution 1 molaire dans le dichlorométhane) comme pour la synthèse de 35. On purifie le composé avec une colonne de chromatographie Séphadex® LH-20 (dichlorométhane/éthanol 1/1), puis sur une colonne de silice (toluène/acétone/éthanol 3/0,5/1) et enfin sur une colonne de silice (diisopropyléther/éthanol 3/2 pour donner le dérivé 37 (116 mg, 44 %).

CCM : Rf = 0,31, gel de silice, éther diisopropylique/acétone/éthanol 3/0,5/0,4 v/v/v

### PRÉPARATION 34

### Méthyl O-(6-O-benzoyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)-]₃-O-(6-O-acétyl-2,3-di-O-méthyl-α-D-glucopyranosyl)-(1→4)-O-(benzyl 2,3-di-O-méthyl-β-D-glucopyranosyl uronate)-(1→4)-O-(3,6-di-O-acétyl-2-O-benzyl-α-D-glucopyranosyl)-(1→4)-O-(benzyl 2,3-di-O-méthyl-α-L-idopyranosyluronate)-(1→4)-2,6-di-O-benzyl-3-O-méthyl-α-D-glucopyranoside (38)

Le composé 37 (110 mg, 0,032 mmol) est transformé en composé 38 (95 mg, 89%) selon le mode opératoire décrit pour la préparation du composé 36.

CCM : Rf = 0,32, gel de silice, toluène/acétone 1/1 v/v

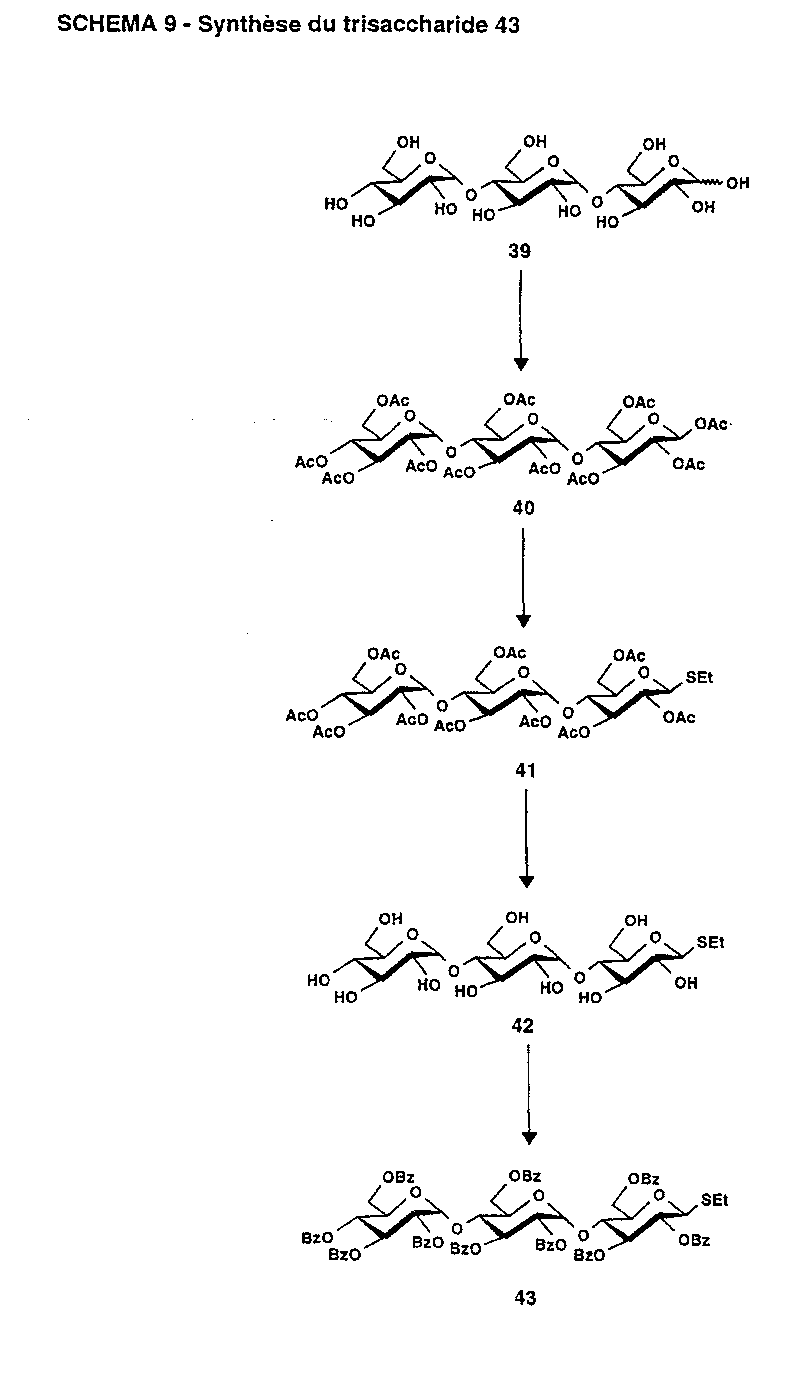

### PRÉPARATION 35

### O-(2,3,4,6-Tétra-O-acétyl-α-D-glucopyranosyl)-(1→4)-O-(2,3,6-tri-O-acétyl-α-D-glucopyranosyl)-(1→4)-1,2,3,6-tétra-O-acétyl-β-D-glucopyranose (40)

On ajoute par petites doses du maltotriose commercial (7 g, 13,9 mmol) à une suspension d'acétate de sodium (7 g, 85 mmol) dans de l'anhydride acétique (70 ml) à 155°C. Après 15 minutes, la solution est refroidie et versée dans de l'eau glacée (700 ml). Après extraction avec de l'acétate d'éthyle, la phase organique est lavée à l'eau, séchée sur sulfate de magnésium, filtrée et concentrée pour donner 13,1 g du composé 40.

CCM : Rf = 0,53, gel de silice, dichlorométhane/acétate d'éthyle 7/3 v/v

### PRÉPARATION 36

### Éthyl O-(2,3,4,6-tétra-O-acétyl-α-D-glucopyranosyl)-(1→4)-O-(2,3,6-tri-O-acétyl-α-D-glucopyranosyl)-(1→4)-2,3,6-tri-O-acétyl-1-thio-β-D-glucopyranoside (41)

Le composé 40 (13 g, 13,5 mmol) est dissous dans du toluène (80 ml). On ajoute sous atmosphère d'azote de l'éthanethiol (1,97 ml, 26,9 mmol) et du diéthylétherate trifluorure de bore (13,7 ml d'une solution molaire dans le toluène). Après 60 heures d'agitation, le mélange est dilué avec de l'eau et du dichlorométhane. Après extraction, la phase organique est lavée avec une solution à 10 % d'hydrogénocarbonate de sodium et de l'eau, séchée, filtrée et concentrée. Le produit brut est purifié par chromatographie sur une colonne de gel de silice pour donner 8,6 g du composé 41.

CCM : Rf = 0,60, gel de silice, dichlorométhane/acétate d'éthyle 7/3 v/v

### PRÉPARATION 37

### Éthyl O-(α-D-glucopyranosyl)-(1→4)-O-(α-D-glucopyranosyl)-(1→4)-1-thio-β-D-glucopyranoside (42)

Le composé 41 est transformé en composé 42 selon le mode opératoire décrit pour la préparation du composé 5.

CCM : Rf = 0,80, gel de silice, acétate d'éthyle/pyridine/acide acétique/eau 13/7/1,6/4 v/v/v/v

### PRÉPARATION 38

### Éthyl O-(2,3,4,6-tétra-O-benzoyl-α-D-glucopyranosyl)-(1→4)-O-(2,3,6-tri-O-benzoyl-α-D-glucopyranosyl)-(1→4)-2,3,6-tri-O-benzoyl-1-thio-β-D-glucopyranoside (43)

Le composé 42 est transformé en composé 43 selon le mode opératoire décrit pour la préparation du composé 2.

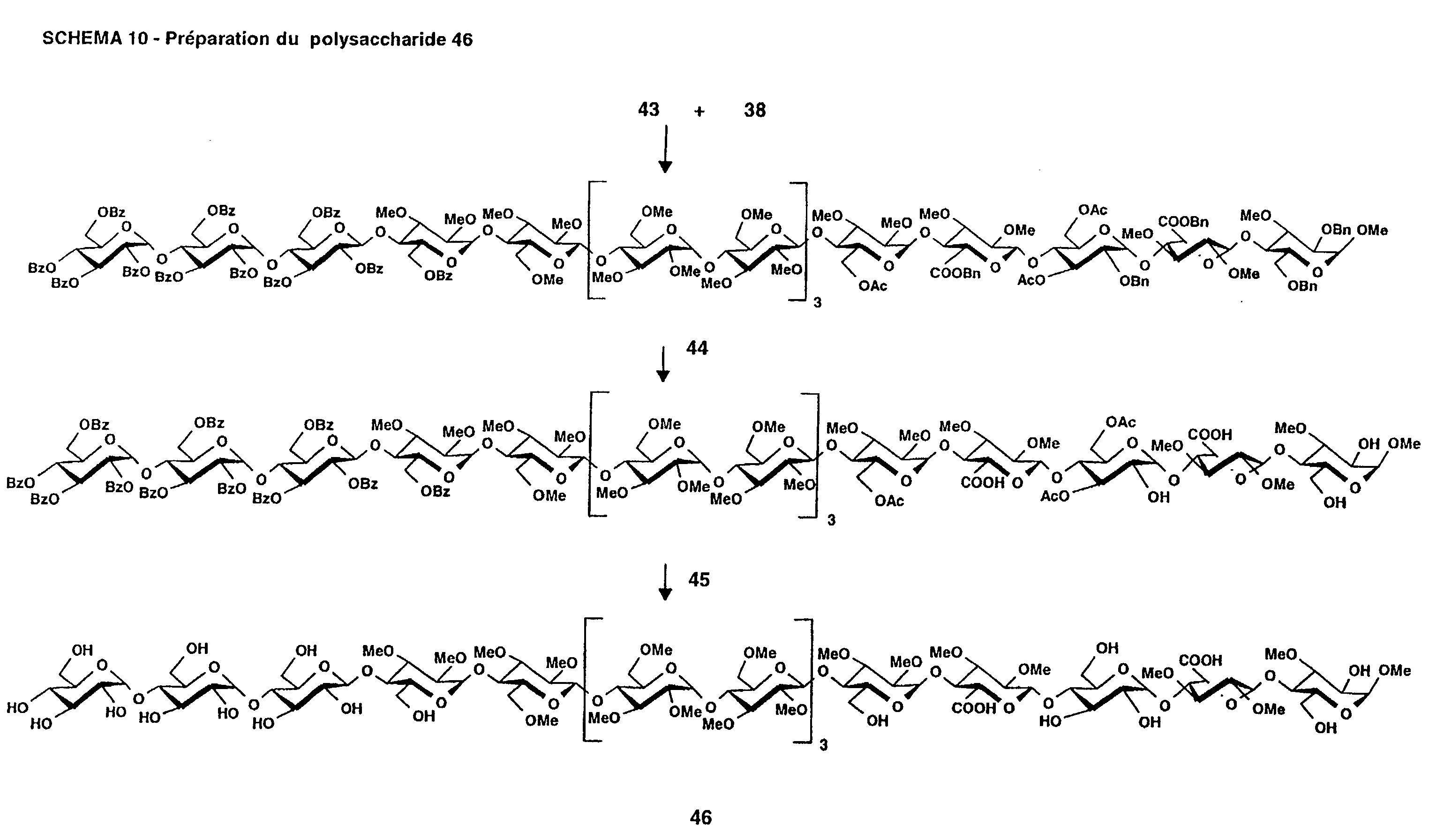

### PRÉPARATION 39

### Méthyl O-(2,3,4,6-tétra-O-benzoyl-α-D-glucopyranosyl)-(1→4)-O-(2,3,6-tri-O-benzoyl-α-D-glucopyranosyl)-(1→4)-O-(2,3,6-tri-O-benzoyl-β-D-glucopyranosyl)-(1→4)-O-(6-O-benzoyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-O-(6-O-acétyl-2,3-di-O-méthyl-α-D-glucopyranosyl)-(1→4)-O-(benzyl 2,3-di-O-méthyl-β-D-glucopyranosyluronate)-(1→4)-O-(3,6-di-O-acétyl-2-O-benzyl-α-D-glucopyranosyl)-(1→4)-O-(benzyl 2,3-di-O-méthyl-α-L-idopyranosyluronate)-(1→4)-2,6-di-O-benzyl-3-O-méthyl-α-D-glucopyranoside (44)

Le thioglycoside 43 (170 mg, 106,7 µmol) et l'accepteur 38 (90 mg, 27 µmol) sont couplés selon le mode opératoire décrit pour le composé 4. Le produit est d'abord purifié par une chromatographie sur Sephadex® LH 20 (dichlorométhane/éthanol 1/1) suivie par une chromatographie sur colonne de gel de silice (cyclohexane/acétone 12/10 v/v) pour donner 89,5 mg (68 %) du composé 44.

CCM : Rf = 0,43, gel de silice, (cyclohexane/acétone 12/10 v/v)

### PRÉPARATION 40

### Méthyl O-(2,3,4,6-tétra-O-benzoyl-α-D-glucopyranosyl)-(1→4)-O-(2,3,6-tri-O-benzoyl-α-D-glucopyranosyl)-(1→4)-O-(2,3,6-tri-O-benzoyl-β-D-glucopyranosyl)-(1→4)-O-(6-O-benzoyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-O-(6-O-acétyl-2,3-di-O-méthyl-α-D-glucopyranosyl)-(1→4)-O-(acide 2,3-di-O-méthyl-β-D-glucopyranosyluronique)-(1→4)-O-(3,6-di-O-acétyl-α-D-glucopyranosyl)-(1→4)-O-(acide 2,3-di-O-méthyl-α-L-idopyranosyluronique)-(1→4)-3-O-méthyl-α-D-glucopyranoside (45)

Une solution du composé 44 (80 mg, 0,0163 mmol) dans de l'acide acétique (3 ml) est traitée sous pression d'hydrogène (10 bar) en présence de palladium sur charbon 10 % (160 mg). Après filtration, la solution est concentrée et conduit au composé 45 qui est utilisé dans l'étape suivante sans purification.

### PRÉPARATION 41

### Méthyl O-(α-D-glucopyranosyl)-(1→4)-O-(α-D-glucopyranosyl)-(1→4)-O-(β-D-gluco pyranosyl)-(1→4)-O-(2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-O-(2,3-di-O-méthyle-α-D-glucopyranosyl)-(1→4)-O-(acide 2,3-di-O-méthyl-β-D-glucopyranosyluronique)-(1→4)-O-(α-D-glucopyranosyl)-(1→4)-O-(acide 2,3-di-O-méthyl-α-L-idopyranosyluronique)-(1→4)-3-O-méthyl-α-D-glucopyranoside (46)

Un mélange de méthanol (2,8 ml) et d'une solution 5 N d'hydroxyde de sodium (0,69 ml) est ajouté au composé 45 (72 mg, 0,016 mmol). On ajoute une solution aqueuse d'hydroxyde de sodium 5 M (en quantité telle que la concentration d'hydroxyde de sodium soit de 0,5 M en fin d'addition) à une solution d'un ester dans le méthanol (150 ml/mmol). Après 2-5 heures on introduit de l'eau et on passe au travers d'une colonne de gel Sephadex® G-25 (1,6 x 115 cm) éluée par l'eau. On concentre, on passe au travers d'une colonne Dowex® 50 H⁺ (2ml) et on lyophilise, A ce stade on vérifie par RMN du proton que tous les groupes protecteurs ont été enlevés. Si cela est nécessaire on soumet de nouveau le produit à l'hydrogénation et/ou la saponification. Le composé 46 (34 mg, 68% en deux étapes) est ainsi obtenu.

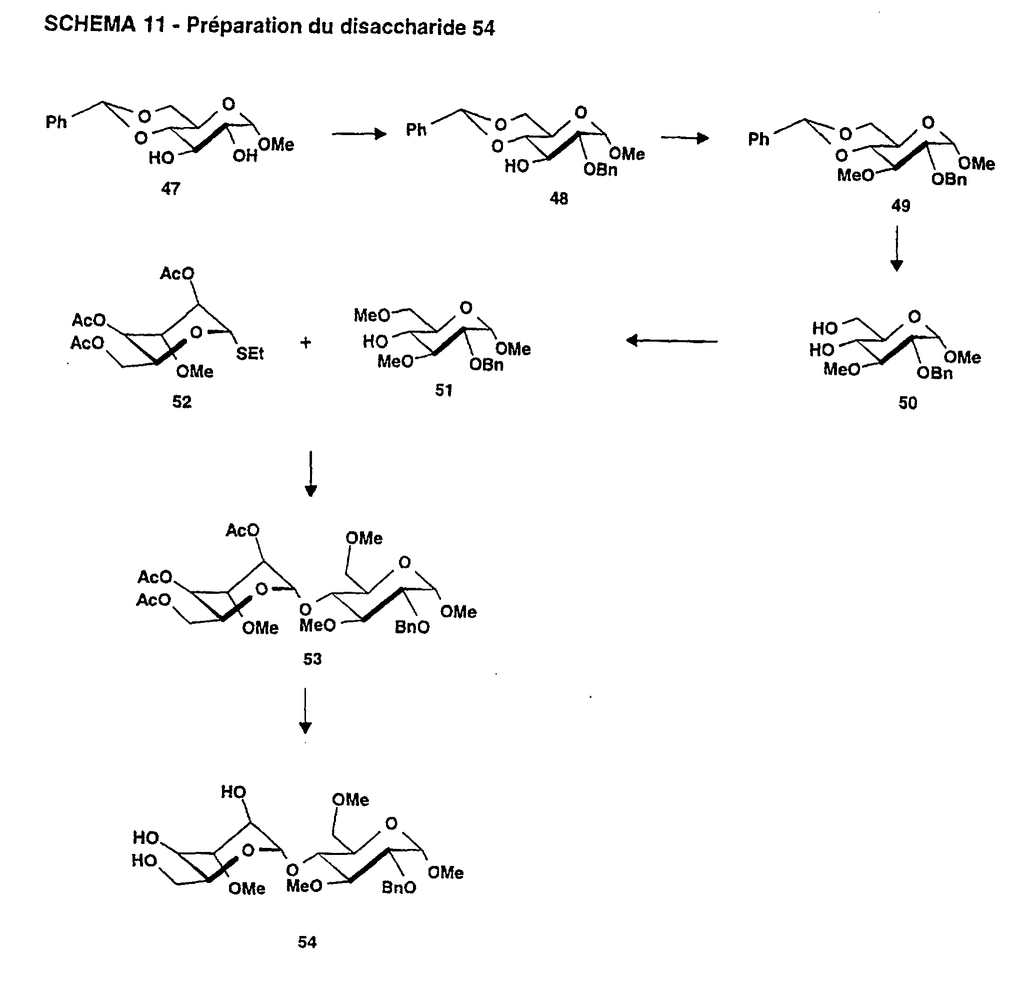

### PRÉPARATION 42

### Méthyl O-4,6-O-benzylidène-2-O-benzyl-α-D-glucopyranoside (48)

Le composé 47 (60 g) (commercialement disponible) est dissous dans du *N*,*N*-diméthylformamide (858 ml) avec du bromure de benzyle (50,5 ml). La solution est refroidie jusqu'à +10 C et une solution aqueuse d'hydroxyde de sodium à 20% est ajoutée goutte à goutte sous agitation du mélange. Après 1 heure, la température réactionnelle est montée à 20° C et le mélange est laissé encore 20 heures sous agitation. La solution est ensuite versée dans un mélange d'eau et de glace et de toluène et extraite. La phase organique est concentrée et le produit brut est purifié par cristallisation pour conduire à 30,0 g du composé 48 décrit par J.M. Küster *et al*. dans Justus Liebigs Ann. Chem. 1975, 2179-2189.

CCM : Rf = 0,60, gel de silice, toluène/acétate d'éthyle 7/3 v/v

### PRÉPARATION 43

### Méthyl O-4,6-O-benzylidène-2-O-benzyl-3-O-méthyl-α-D-glucopyranoside (49)

Le composé 48 (26,4 g) est dissous dans du *N*,*N*-diméthylformamide (211 ml) et refroidi à +5°C. De l'hydrure de sodium (3,2g) est ajouté sous atmosphère d'azote. Puis de l'iodométhane (11,3 g) est ajouté goutte à goutte et le mélange est agité pendant 1 heure à température ambiante. Le mélange est dilué dans de l'acétate d'éthyle, lavé 2 fois à l'eau et concentré pour conduire à 28 g du composé 49 brut décrit par M. Petitou *et al*. dans J. Med. Chem. 1997, *40*, 1600-1607.

CCM : Rf = 0,70, gel de silice, (toluène/acétate d'éthyle 7/3 v/v).

### PRÉPARATION 44

### Méthyl O-2-O-benzyl-3-O-méthyl-α-D-glucopyranoside (50)

Le composé 49 (28 g) est dissous dans du méthanol (468 ml). De l'acide *p*-toluène sulfonique (1,35 g) est ajouté et le mélange est agité pendant 20 heures à 20° C. Le mélange est dilué avec du toluène et concentré. Une purification par chromatographie sur une colonne de gel de silice conduit à 21 g du composé 50.

CCM : Rf = 0,07, gel de silice, (toluène/acétate d'éthyle 6/4 v/v).

### PRÉPARATION 45

### Méthyl O-2-O-benzyl-3,6-di-O-méthyl-α-D-glucopyranoside (51)

Le composé 50 (11.8 g) est dissous dans du dichlorométhane (160 ml) sous atmosphère d'azote. Du tétrafluoroborate de triméthyloxonium (7,0 g) et de la 2,6-di-*tert*-butyl-4-méthylpyridine (10,6 g) sont ajoutés à température ambiante. Après 2 heures, le mélange est versé dans un mélange d'eau et de glace et extrait avec du dichlorométhane. La phase organique est lavée avec de l'hydrogénocarbonate de sodium et concentrée. La purification du produit brut par chromatographie sur gel de silice conduit à 10,1 g du composé 51.

CCM : Rf = 0,25, gel de silice, (toluène/acétate d'éthyle 7/3 v/v).

### PRÉPARATION 46

### Ethyl 2,4,6-tri-O-acétyl-3-O-méthyl-1-thio-α-L-idopyranose (52)

Du 1,2,4,6-tétra-O-acétyl-3-O-méthyl-α-L-idopyranose (préparé tel que décrit pour son analogue *per*-benzoylé en remplaçant le chlorure de benzoyle par de l'anhydride acétique ; Jaurand *et al* Bio. Med. Chem. Lett. 1992, 2, 897-900) (48,4 g) est dissous dans du toluène (175 ml). De l'éthanethiol (20 ml) et de du trifluorure de bore-éthérate (1M dans le toluène, 134 ml) sont ajoutés sous atmosphère d'azote. Après 1 heure sous agitation, de l'hydrogénocarbonate de sodium aqueux (400 ml) et le mélange est ajouté et le mélange est agité pendant 1 heure. Le mélange est ensuite versé dans de l'acétate d'éthyle. La phase organique est lavée deux fois avec de l'eau et concentrée. La purification par chromatographie sur colonne de gel de silice conduit à 29,6 g du composé 52.

CCM : Rf = 0,45, gel de silice, (toluène/acétate d'éthyle 6/4 v/v).

### PRÉPARATION 47

### Méthyl O-(2,4,6-tri-O-acétyl-3-O-méthyl-α-L-idopyranosyl)-(1→4)-2-O-benzyl-3,6-di-O-méthyl-6-O-méthyl-α-D-glucopyranoside (53)

Le composé 51 (10,1 g) et le composé 52 (16,0 g) sont dissous dans du toluène (304 ml) sous atmosphère d'azote. Après addition de tamis moléculaire pulvérulent (4Å) le milieu réactionnel est refroidi à -20°C. Une solution 0,1M fraîchement préparée de N-iodosuccinimide (10,1 g) et d'acide trifluorométhanesulfonique (0,80 ml) dans un mélange dioxane/dichlorométhane 1/1 v/v est ajoutée goutte à goutte sous un courant d'azote. Après 10 minutes, le mélange réactionnel, rouge, est filtré et lavé successivement avec du thiosulfate de sodium aqueux et de l'hydrogénocarbonate de sodium aqueux. La phase organique est concentrée et le résidu est purifié par chromatographie sur gel de silice pour conduire à 21,1 g du composé 53.

CCM : Rf = 0,30, gel de silice, (toluène/acétate d'éthyle 6/4 v/v).

### PRÉPARATION 48

### Méthyl O-(3-O-méthyl-α-L-idopyranosyl)-(1→4)-2-O-benzyl-3,6-di-O-méthyl-α-D-glucopyranoside (54)

Le composé 53 (21,1 g) est dissous dans 295 ml d'un mélange méthanol/dioxane (1/1 v/v) et du *tert*-butylate de potassium est ajouté. Après 30 minutes, le mélange est neutralisé avec une résine Dowex® 50WX8 sous forme H⁺ et concentré sous vide. La purification est réalisée par chromatographie sur gel de silice pour conduire à 12,7 g du composé 54.

CCM : Rf = 0,08, gel de silice, (toluène/acétate d'éthyle 3/7 v/v)

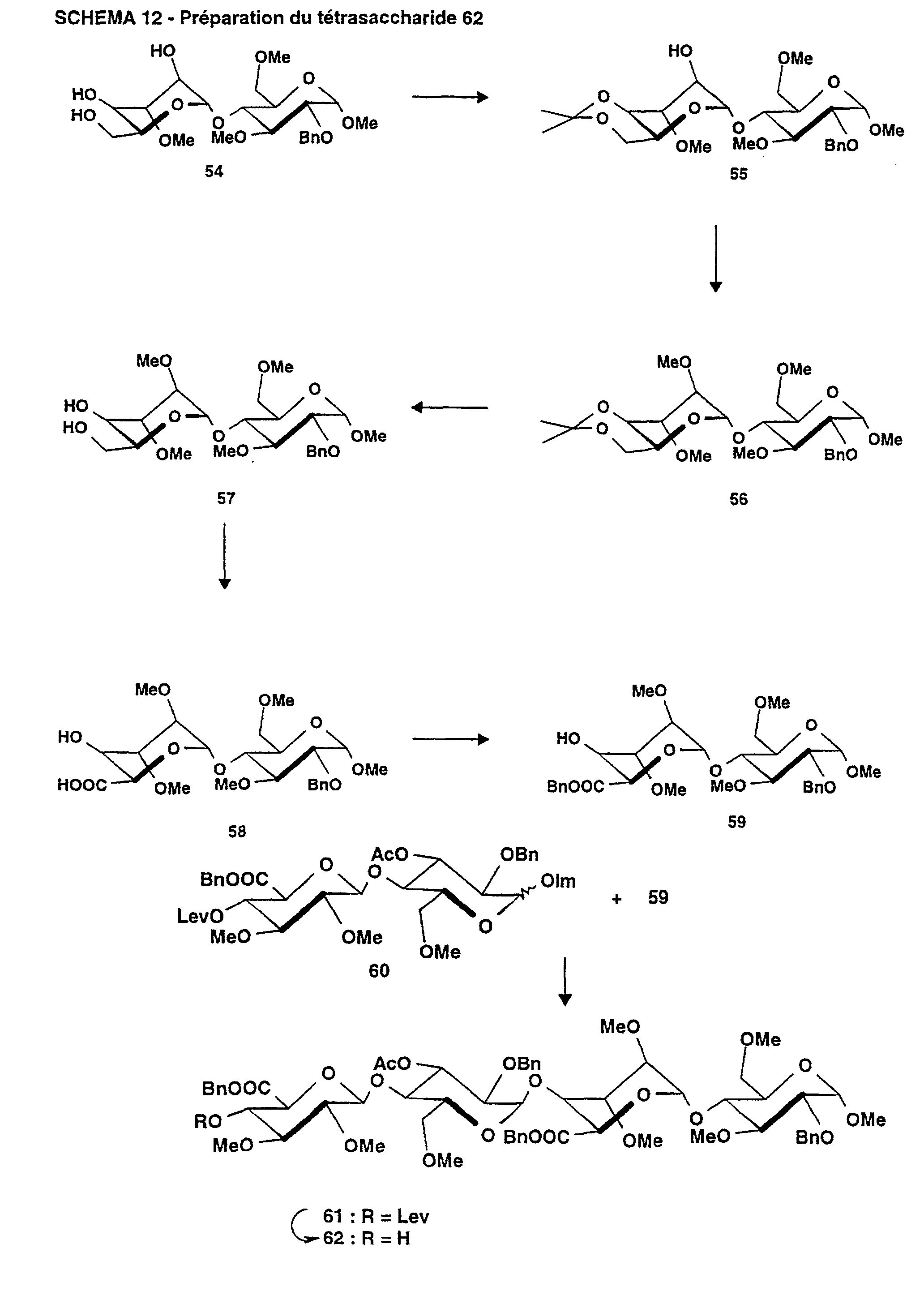

### PRÉPARATION 49

### Méthyl O-(4,6-O-isopropylidène-3-O-méthyl-α-L-idopyranosyl)-(1→4)-2-O-benzyl-3,6-di-O-méthyl-α-D-glucopyranoside (55)

Le composé 54 (12,7 g) est dissous dans du tétrahydrofurane (67 ml) sous atmosphère d'azote. Du 2,2-diméthoxypropane (19 ml) et de l'acide *p*-toluènesulfonique sont ajoutés et le mélange est ensuite agité pendant 16 heures à température ambiante. Le milieu réactionnel est ensuite dilué avec de l'hydrogénocarbonate de sodium aqueux et extrait avec de l'acétate d'éthyle pour conduire après évaporation du solvant à un résidu brut qui est purifié par chromatographie sur gel de silice pour donner 10,4 g du composé 55.

CCM : Rf = 0,35, gel de silice, (toluène/acétate d'éthyle 1/1 v/v).

### PRÉPARATION 50

### Méthyl O-(4,6-O-isopropylidène-2,3-di-O-méthyl-α-L-idopyranosyl)-(1→4)-2-O-benzyl-3,6-di-O-méthyl-α-D-glucopyranoside (56)

Le composé 55 (10,4g) est dissous dans du trétrahydrofurane (140 ml) et refroidi à 10° C. De l'hydrure de sodium (1,38 g ; dispersion à 60% dans l'huile) et de l'iodométhane (1,84 ml) sont ajoutés sous atmosphère d'azote. Après 4 heures, l'excès d'hydrure de sodium est détruit avec du méthanol et le mélange est extrait avec du dichlorométhane et concentré. Le résidu est purifié par chromatographie sur gel de silice pour conduire à 11,1 g du composé 56.

CCM : Rf = 0,55, gel de silice, (toluène/acétate d'éthyle 95/5 v/v).

### PRÉPARATION 51

### Méthyl O-(2,3-di-O-méthyl-α-L-idopyranosyl)-(1→4)-2-O-benzyl-3,6-di-O-méthyl-α-D-glucopyranoside (57)

Le composé 56 (11,1 g) est dissous dans un mélange acide acétique/eau 7/3 (v/v) et agité une nuit. Le mélange est évaporé deux fois en présence de toluène et purifié par chromatographie sur gel de silice pour conduire à 9,2 g du composé 57.

CCM : Rf = 0,09, gel de silice, (toluène/acétate d'éthyle 1/1 v/v).

### PRÉPARATION 52

### Méthyl O-(acide 2,3-di-O-méthyl-α-L-idopyranosyluronique)-(1→4)-2-O-benzyl-3,6-di-O-méthyl-α-D-glucopyranoside (58)

Du 2,2,6,6-tétraméthyl-1-pipéridinyloxy (13 mg), une solution saturée d'hydrogénocarbonate de sodium (16 ml), du bromure de potassium (88 mg) et du chlorure de tétrabutylammonium (117 mg) sont ajoutés à une solution du composé 57 (4,7 g) dans du dichlorométhane (28 ml). Le mélange est refroidi à 0°C et un mélange d'une solution saturée de chlorure de sodium (17,8 ml), d'une solution saturée d'hydrogénocarbonate de sodium (8,8 ml) et de l'hypochlorite de sodium (1,3 M; 20 ml) est ajoutée pendant 15 minutes. Après 1 heure sous agitation, le milieu est dilué avec de l'acétate d'éthyle, lavé avec de l'eau saturée en chlorure de sodium, séché sur sulfate de magnésium, filtré et évaporé à sec pour conduire à 3,5 g du composé brut 58.

CCM : Rf = 0,14, gel de silice, dichlorométhane /méthanol 9/1 v/v

### PRÉPARATION 53

### Méthyl O-(benzyl-2,3-di-O-méthyl-α-L-idopyranosyluronate)-(1→4)-2-O-benzyl-3,6-di-O-méthyl-α-D-glucopyranoside (59)

Le composé 58 (3,5 g) est dissous dans du *N*,*N*-diméthylformamide (29 ml) sous atmosphère d'azote. De l'hydrogénocarbonate de potassium (1,76 g) et du bromure de benzyle (2,85 ml) sont ajoutés et le mélange est agité pendant 4 heures. De l'acétate d'éthyle et de l'eau sont ajoutés et la phase organique est concentrée après extraction. La purification par chromatographie sur gel de silice conduit à 3,4 g du composé 59.

CCM : Rf = 0,43, gel de silice, toluène/acétate d'éthyl 4/6 v/v

### PRÉPARATION 54

### Méthyl O-(benzyl 4-O-lévulinoyl-2,3-di-O-méthyl-β-D-glucopyranosyluronate)-(1→4)-O-(3-O-acétyl-2-O-benzyl-6-O-méthyl-α-D-glucopyranosyl)-(1→4)-O-(benzyl 2,3-di-O-méthyl-α-L-idopyranosyluronate)-(1→4)-2-O-benzyl-3,6-di-O-méthyl-α-D-glucopyranoside (61)

Un mélange du composé 59 (0,53 g) et du composé 60 (1,0 g) est évaporé en présence de toluène et dissous dans du dichlorométhane (16,2 ml) sous atmosphère d'azote. Après addition de tamis moléculaire pulvérulent (4Å), le mélange est refroidi à -20°C. Après agitation pendant 15 minutes du trifluorométhanesulfonate de triméthylsilyl (15% en mol par rapport au composé 60) est ajouté. Après 15 minutes, le mélange est traité avec de l'hydrogénocarbonate de sodium aqueux. Après filtration du tamis moléculaire, le filtrat est dilué avec du dichlorométhane, lavé à l'eau, concentré et purifié par chromatographie sur gel de silice pour conduire à 0,75 g du composé 61.

RMN : Rf = 0,45, gel de silice, (toluène/acétate d'éthyle3/7 v/v).

### PRÉPARATION 55

### Méthyl O-(benzyl 2,3-di-O-méthyl-β-D-glucopyranosyluronate)-(1→4)-O-(3-O-acétyl-2-O-benzyl-6-O-méthyl-α-D-glucopyranosyl)-(1→4)-O-(benzyl-2,3-di-O-méthyl-α-L-idopyranosyluronate)-(1→4)-2-O-benzyl-3,6-di-O-méthyl-α-D-glucopyranoside (62).

Le composé 61 (0,74 g) est dissous dans de la pyridine (3,0 ml) et un mélange d'acide acétique (4,1 ml), d'hydrate d'hydrazine (0,47 ml) dans de la pyridine (3,0 ml) est ajouté à température ambiante. Après 9 heures sous agitation, du dichlorométhane et de l'eau sont ajoutés. La phase organique est séparée et lavée successivement avec une solution d'acide chlorhydrique (1,0 N), d'hydrogénocarbonate de sodium aqueux et de l'eau. La phase organique est concentrée, purifiée par chromatographie sur gel de silice pour conduire à 0,66 g du composé 62.

CCM : Rf = 0,25, gel de silice, (dichlorométhane /méthanol 98/2 v/v).

La préparation du tétrasaccharide 68 ci-dessous est réalisée de façon analogue en suivant le schéma réactionnel 13 ci-après à partir du disaccharide 55.

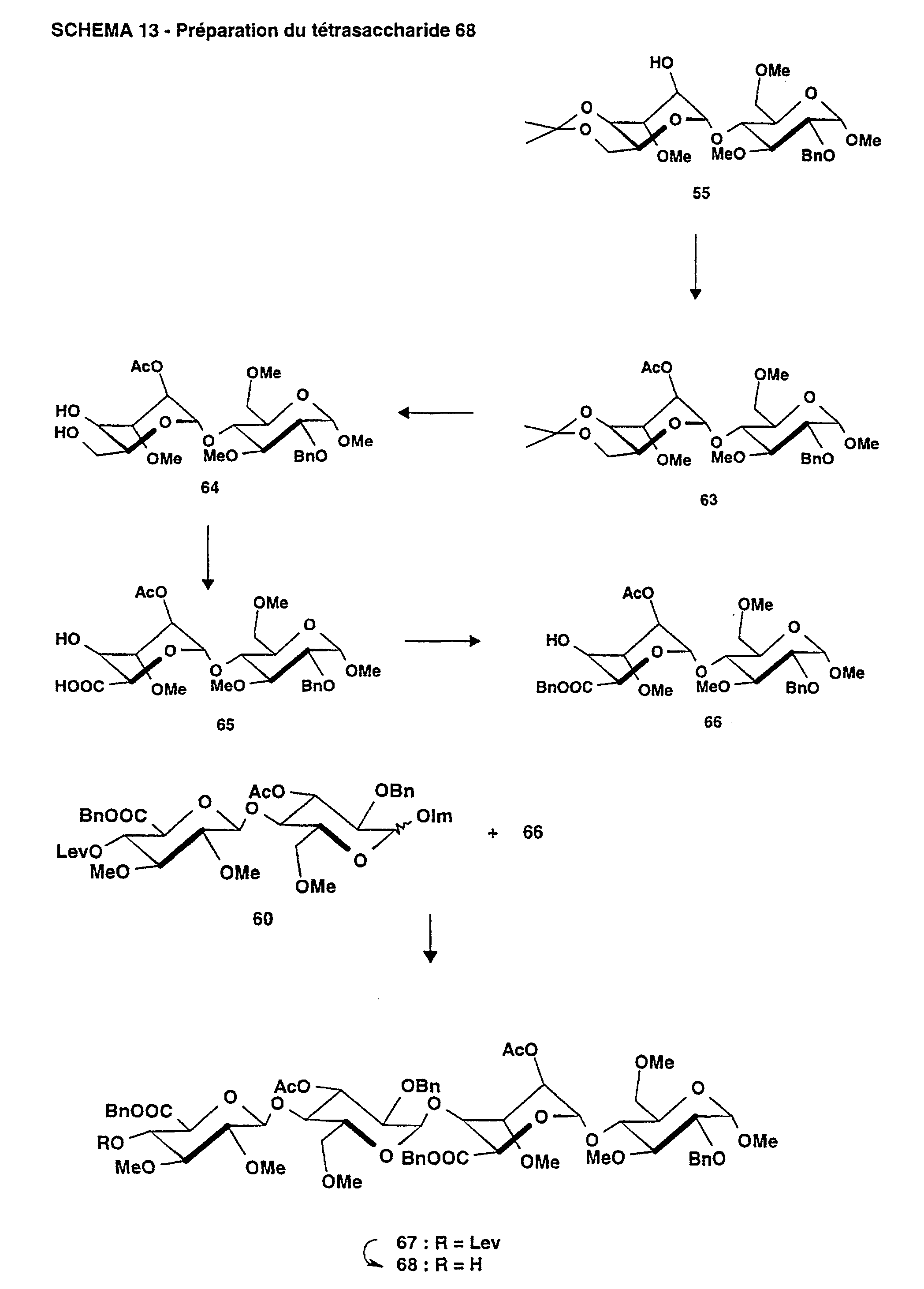

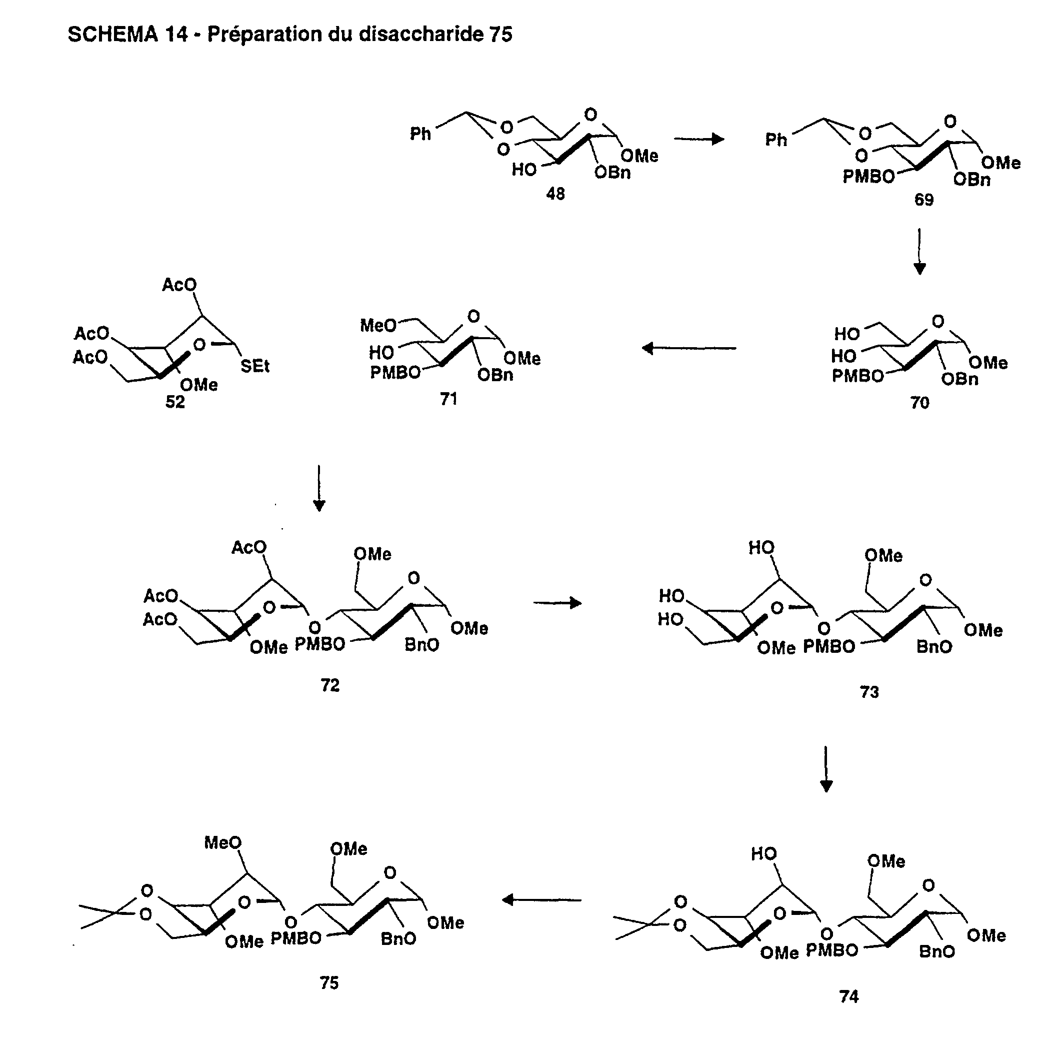

### PRÉPARATION 56

### Méthyl 2-O-benzyl-4,6-di-O-benzylidène-3-O-p-méthoxybenzyl-α-D-glucopyranoside (69)

Le composé 48 (26,4 g) est dissous dans du *N*,*N*-diméthylformamide (211 ml) et refroidi à 5°C. De l'hydrure de sodium (2,5 g) est ajouté sous atmosphère d'azote. Puis on ajoute goutte à goutte du chlorure de 4-méthoxybenzyle (13,3 g) et le mélange est agité pendant 1 heure à température ambiante. Le mélange est dilué avec de l'acétate d'éthyle, lavé deux fois avec de l'eau et concentré pour donner 40,7 g du composé 69.

CCM : Rf = 0,80, gel de silice, toluène/acétate d'éthyle 7/3 v/v

### PRÉPARATION 57

### Méthyl O-benzyl 3-O-p-méthoxybenzyl-α-D-glucopyranoside (70)

Le composé 69 (34,9 g) est dissous dans l'acide acétique aqueux à 60% et agité pendant 4 heures à 60°C. Le mélange est dilué avec du toluène et concentré. La purification par chromatographie sur une colonne de gel de silice permet d'obtenir 26,4 g du composé 70.

CCM : Rf = 0,07, gel de silice, toluène/acétate d'éthyle 7/3 v/v

### PRÉPARATION 58

### Méthyl 2-O-benzyl-3-O-p-méthoxybenzyl-6-O-méthyl-α-D-glucopyranoside (71)

Le composé 70 (26,4 g) est dissous dans du dichlorométhane (263 ml) sous atmosphère d'azote. On ajoute à température ambiante du triméthyloxoniumtétrafluoroborate (11,6 g ) et du 2,6-di-*tert*-butyl-4-méthylpyridine (17,4 g). Après 4 heures, le mélange est versé sur de l'eau glacée et extrait avec du dichlorométhane. La phase organique est lavée avec de l'hydrogénocarbonate de sodium et concentrée. La purification du produit brut par une chromatographie sur colonne de gel de silice permet d'obtenir 18,5 g du composé 71.

CCM : Rf = 0,25, gel de silice, toluène/acétate d'éthyle 7/3 v/v

### PRÉPARATION 59

### Méthyl O-(2,4,6-tri-O-acétyl-3-O-méthyl-α-L-idopyranosyl)-(1→4)-2-O-benzyl-3-O-p-méthoxybenzyl-6-O-méthyl-α-D-glucopyranoside (72)

Le composé 71 (17,5 g) et le composé 52 (28,2 g) sont dissous dans le toluène (525 ml) sous atmosphère d'azote. Après l'ajout d'un tamis moléculaire 4Å, la réaction est refroidie à -20°C. Une solution de 0,1 M de *N*-iodosuccinimide (17,4 g) fraîchement préparée et d'acide trifluorométhanesulfonique (1,38 ml) dans le dioxane/dichlorométhane 1/1 v/v est ajoutée goutte à goutte sous un flux continu d'azote. Après 10 minutes, le mélange réactionnel rouge est filtré et lavé successivement avec du thiosulfate de sodium aqueux et de l'hydrogénocarbonate de sodium aqueux. La phase organique est concentrée sous vide et 30,0 g du composé 72 sont isolés.

CCM : Rf = 0,45, gel de silice, dichlorométhane /acétate d'éthyle 8/2 v/v

### PRÉPARATION 60

### Méthyl O-(3-O-méthyl-α-L-idopyranosyl)-(1→4)-2-O-benzyl-3-O-p-méthoxybenzyl-6-O-méthyl-α-D-glucopyranoside (73)

Le composé 72 (30,0 g) est dissous dans 460 ml de méthanol/dioxane 1/1 v/v et on ajoute du *tert*-butylate de potassium. Après 15 minutes, le mélange est neutralisé avec une résine Dowex® 50WX8H⁺ et concentré sous vide. La purification est réalisée par chromatographie sur colonne de gel de silice pour donner 17,4 g du composé 73.

CCM : Rf = 0,25, gel de silice, dichlorométhane /méthanol 95/5 v/v

### PRÉPARATION 61

### Méthyl O-(4,6-O-isopropylidène-3-O-méthyl-α-L-idopyranosyl)-(1→4)-2-O-benzyl-3-O-p-méthoxybenzyl-6-O-méthyl-α-D-glucopyranoside (74)

Sous atmosphère d'azote, le composé 73 (17,4 g) est dissous dans du *N*,*N*-diméthylformamide (77 ml). On ajoute du 2,2-diméthoxypropane (26 ml) et de l'acide *p*-toluènesulfonique et le mélange est ensuite agité pendant 30 minutes. La dilution du mélange avec de l'hydrogénocarbonate de sodium aqueux puis son extraction avec de l'acétate d'éthyle permet d'obtenir 19,7 g du composé 74 après évaporation du solvant.

CCM : Rf = 0,45, gel de silice, dichlorométhane /méthanol 95/5 v/v

### PRÉPARATION 62

### Méthyl O-(4,6-O-isopropylidène-2,3-di-O-méthyl-α-L-idopyranosyl)-(1→4)-2-O-benzyl-6-O-méthyl-α-D-glucopyranoside (75)

Le composé 74 (18,5 g) est dissous dans du *N*,*N*-diméthylformamide (24,4 ml) et refroidi à 0°C. Sous atmosphère d'azote, on ajoute de l'hydrure de sodium (1,47 g ; dispersion dans l'huile 60%) et de l'iodométhane (2,36 ml). Après 1 heure, l'excès d'hydrure de sodium est détruit avec du méthanol et le mélange est extrait avec du dichlorométhane et concentré pour donner 20,0 g du 75.

CCM : Rf = 0,85, gel de silice, dichlorométhane /méthanol 95/5 v/v.

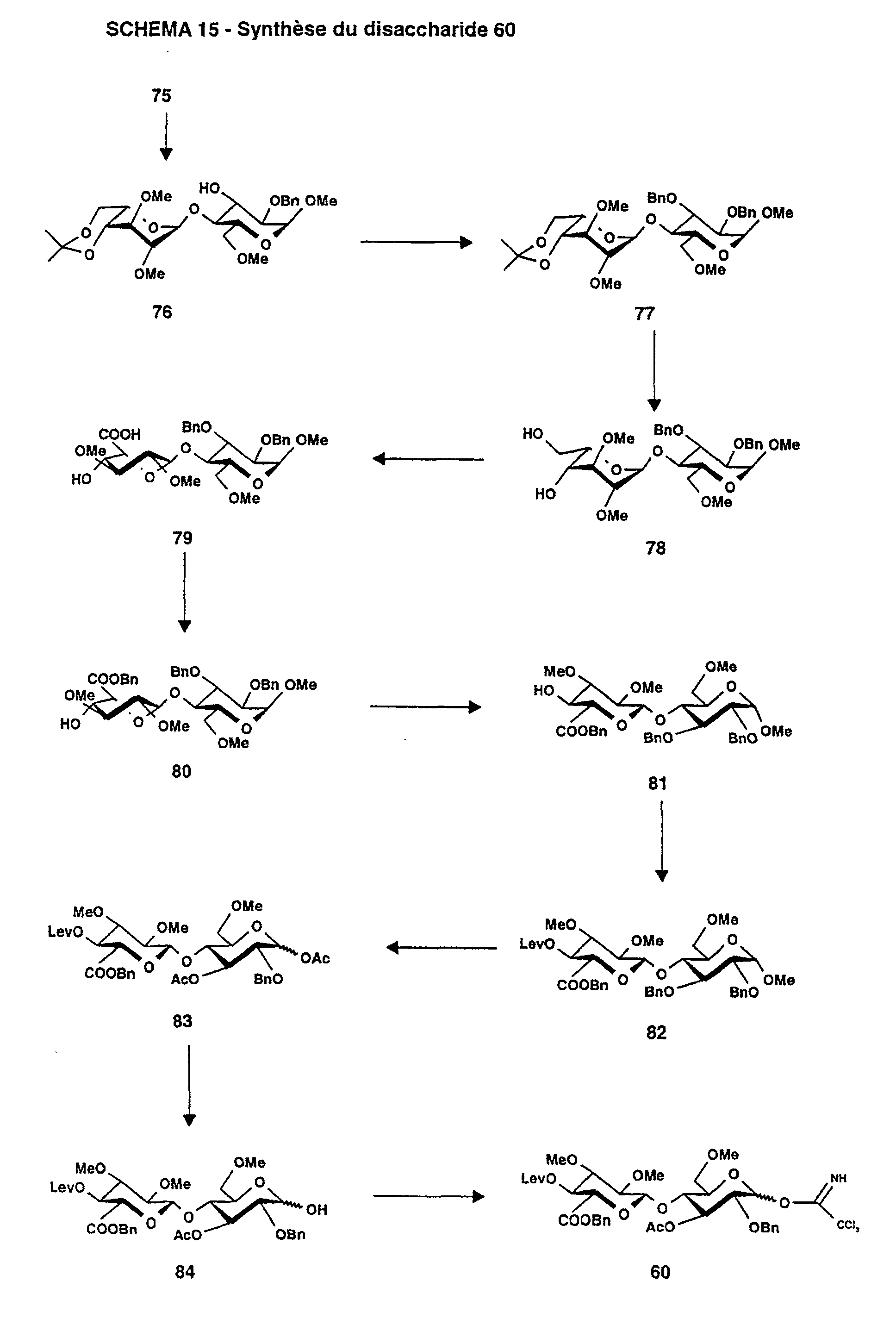

### PRÉPARATION 63

### Méthyl O-(4,6-O-isopropylidène-2,3-di-O-méthyl-α-L-idopyranosyl)-(1→4)-2-O-benzyl-6-O-méthyl-a-D-glucopyranoside (76)

Le composé 75 (18,4 g) est dissous dans du dichlorométhane (838 ml) et de l'eau (168 ml). On ajoute de la 2,3-dichloro-5,6-dicyano-1,4-benzoquinone (7,1 g) et le mélange est agité pendant 18 heures à 4°C. Le mélange est versé dans de l'hydrogénocarbonate de sodium aqueux et extrait au dichlorométhane. La concentration de la phase organique fournit 12,7 g du compose 76.

CCM : Rf = 0,40, gel de silice, dichlorométhane /méthanol 95/5 v/v.

### PRÉPARATION 64

### Méthyl O-(4,6-O-isopropylidène-2,3-di-O-méthyl-α-L--idopyranosyl)-(1→4)-2,3-di-O-benzyl-6-O-méthyl-α-D-glucopyranoside (77)

Le composé 76 (10,5 g) est dissous dans du *N*,*N*-diméthylformamide sec (178 ml) puis refroidi à 0°C sous atmosphère d'azote. On ajoute de l'hydrure de sodium (1,91 g ; dispersion dans l'huile 60%) puis goutte à goutte du bromure de benzyle (3,3 ml). Après 30 minutes, la réaction est terminée et l'excès d'hydrure de sodium est détruit avec du méthanol. On ajoute de l'eau et le mélange est extrait deux fois avec de l'acétate d'éthyle. L'évaporation du solvant permet d'obtenir 13,6 g du composé 77.

CCM : Rf = 0,50, gel de silice, toluène/acétate d'éthyle 1/1 v/v.

### PRÉPARATION 65

### Méthyl O-(2,3-di-O-méthyl-α-L-idopyranosyl)-(1→4)-2,3-di-O-benzyl-6-O-méthyl-α-D-glucopyranoside (78).

Le composé 77 est dissous dans de l'acide acétique/eau 77/33 (v/v) et agité pendant une nuit. Le mélange est co-évaporé deux fois avec du toluène et purifié par chromatographie sur colonne de gel de silice pour obtenir 11,5 g du composé 78.

CCM : Rf = 0,09, gel de silice, toluène/acétate d'éthyle 1/1 v/v.
Rf = 0,68, gel de silice, dichlorométhane/méthanol 9/1 v/v.

### PRÉPARATION 66

### Méthyl O-(acide 2,3-di-O-méthyl-α-L-idopyranosyluronique)-(1→4)-2,3-di-O-benzyl-6-O-méthyl-α-D-glucopyranoside (79).

A une solution du composé 78 (11,6 g) dans du dichlorométhane (60 ml), on ajoute du 2,2,6,6-tétraméthyl-1-pipéridinyloxy (33 mg), une solution d'hydrogénocarbonate de sodium (40 ml), du bromure de potassium (218 mg) et du chlorure de tétrabutyl ammonium (289 mg). Le mélange est refroidi à 0°C et on ajoute pendant 15 minutes un mélange d'une solution saturée de chlorure de sodium (44 ml), d'une solution saturée d'hydrogénocarbonate de sodium (21,8 ml) et d'hypochlorite de sodium (1,3 M, 50 ml). Après agitation pendant 1 heure, le mélange est dilué avec de l'eau et extrait (3 fois) avec du dichlorométhane. La phase organique est lavée avec une solution aqueuse de chlorure de sodium, séchée sur sulfate de magnésium, filtrée et évaporée à sec pour donner 13,4 g du composé brut 79.

CCM : Rf = 0,14, gel de silice, dichlorométhane /méthanol 9/1 v/v.

### PRÉPARATION 67

### Méthyl O-(benzyl 2,3-di-O-méthyl-α-D-idopyranosyluronate)-(1→4)-2,3-di-O-benzyl-6-O-méthyl-α-D-glucopyranoside (80).

Sous atmosphère d'azote le composé 79 est dissous dans du *N*,*N*-diméthylformamide (110 ml). On ajoute de l'hydrogénocarbonate de potassium (6,7 g) et du bromure de benzyle (10,7 ml) et le mélange est agité pendant 90 minutes. On ajoute de l'acétate d'éthyle et de l'eau puis après extraction, la phase organique est concentrée. La purification par chromatographie sur une colonne de gel de silice permet d'obtenir 9,9 g du composé 80.

CCM : Rf = 0,43, gel de silice, toluène/acétate d'éthyle 4/6 v/v.

### PRÉPARATION 68

### Méthyl O-(benzyl 2,3-di-O-méthyl-β-D-glucopyranosyluronate)-(1→4)-2,3-di-O-benzyl-6-O-méthyl-α-D-glucopyranoside (81).

Le composé 80 (9,9 g) est dissous dans 300 ml de méthanol et chauffé à reflux sous atmosphère d'azote. On ajoute goutte à goutte 1M de méthylate de sodium dans du méthanol (65,2 ml) et le mélange est agité et chauffé à reflux pendant 3 heures. Le mélange est ensuite refroidi à température ambiante, on ajoute de l'hydroxyde de sodium 1N (22,2 ml) et le mélange réactionnel est agité pendant encore 90 minutes. Après neutralisation avec la résine Dowex® 50WX8 H⁺ et filtration, le mélange est concentré. Le produit pur est dissous dans du *N*,*N*-diméthylformamide (192 ml) et un tamis moléculaire est ajouté sous atmosphère d'azote. On ajoute de l'hydrogénocarbonate de potassium (3,2 g) et du bromure de benzyle (4,8 ml) et le mélange est agité pendant 5 heures. Après addition d'acétate d'éthyle et d'eau, extraction et séparation des deux phases, la phase organique est concentrée. Le produit brut est purifié par chromatographie sur colonne de gel de silice pour donner 6,19 g du composé 81 et 1,88 g du composé 80 de départ.

CCM : Rf = 0,55, gel de silice, toluène/acétate d'éthyle 4/6 v/v.

### PRÉPARATION 69

### Méthyl O-(benzyl-4-O-lévulinoyl-2,3-di-O-méthyl-β-D-glucopyranosyluronate)-(1→4)-2,3-di-O-benzyl-6-O-méthyl-α-D-glucopyranoside (82).

Le composé 81 (6,2 g) est dissous dans 40 ml de dioxane. On ajoute de l'acide lévulinique (2,1 g), du dicyclohexylcarbodiimide (3,75 g) et de la 4-diméthylaminopyridine (0,2 g) et le mélange est agité pendant 2 heures sous atmosphère d'azote. On ajoute de l'éther diéthylique (95 ml) et le précipité est filtré. Le filtrat est lavé avec de l'hydrogénosulfate de potassium aqueux, séché sur sulfate de magnésium, filtré et concentré. La cristallisation dans éther/heptane permet d'obtenir 6,2 g du composé 82.

CCM : Rf = 0,26, gel de silice, dichlorométhane /acétone 95/5 v/v.

### PRÉPARATION 70

### O-(Benzyl-4-O-lévulinoyl-2,3-di-O-méthyl-β-D-glucopyranosyluronate)-(1→4)-1,3-di-O-acétyl-2-O-benzyl-6-O-méthyl-α,β-D-glucopyranose (83).

Le composé 82 (6,1 g) est dissous dans de l'anhydre acétique (256 ml) sous atmosphère d'azote et refroidi à -20°C. Un mélange d'acide sulfurique (4,9 ml) dans de l'anhydride acétique (49 ml) est ajouté goutte à goutte pendant 30 minutes. Après 60 minutes, on ajoute de l'acétate de sodium jusqu'à obtention d'un mélange ayant un pH neutre. On ajoute alors de l'acétate d'éthyle et de l'eau et la phase organique est concentrée. La purification par chromatographie sur une colonne de gel de silice permet d'obtenir 4,2 g du composé 83.

CCM : Rf = 0,24, gel de silice, dichlorométhane /acétate d'éthyle 8/2 v/v.

### PRÉPARATION 71

### O-(Benzyl 4-O-lévulinoyl-2,3-di-O-méthyl-β-D-glucopyranosyluronate)-(1→4)-3-O-acétyl-2-O-benzyl-6-O-méthyl-α,β-D-glucopyranose (84).

Le composé 83 (4,2 g) est dissous dans du trétrahydrofurane (42 ml) et on ajoute de la pipéridine (4,1 ml). Le mélange est agité pendant la nuit à température ambiante. On ajoute de l'acétate d'éthyle et le mélange est lavé avec de l'acide chlorhydrique 0,5 N. La phase organique est concentrée et le résidu purifié par chromatographie sur une colonne de gel de silice pour donner 3,2 g du composé 84.

CCM : Rf = 0,33, gel de silice, dichlorométhane /acétate d'éthyle 1/1 v/v.

### PRÉPARATION 72

### O-(Benzyl 4-O-lévulinoyl-2,3-di-O-méthyl-a-D-glucopyranosyluronate)-(1→4)-3-O-acétyl-2-O-benzyl-6-O-méthyl-α,β-D-glucopyranose trichloroacétimidate (60)

Le composé 84 (1,59 g) est dissous dans du dichlorométhane sec sous atmosphère d'azote. On ajoute du trichloroacétonitrile (1,1 ml) et du carbonate de césium (72 mg) et le mélange est agité pendant 1 heure. Le carbonate de césium est filtré et le filtrat est concentré. La purification par chromatographie sur colonne de gel de silice permet d'obtenir 1,57 g du composé 60.

CCM : Rf = 0,60, gel de silice, toluène/acétate d'éthyle 3/7 v/v.

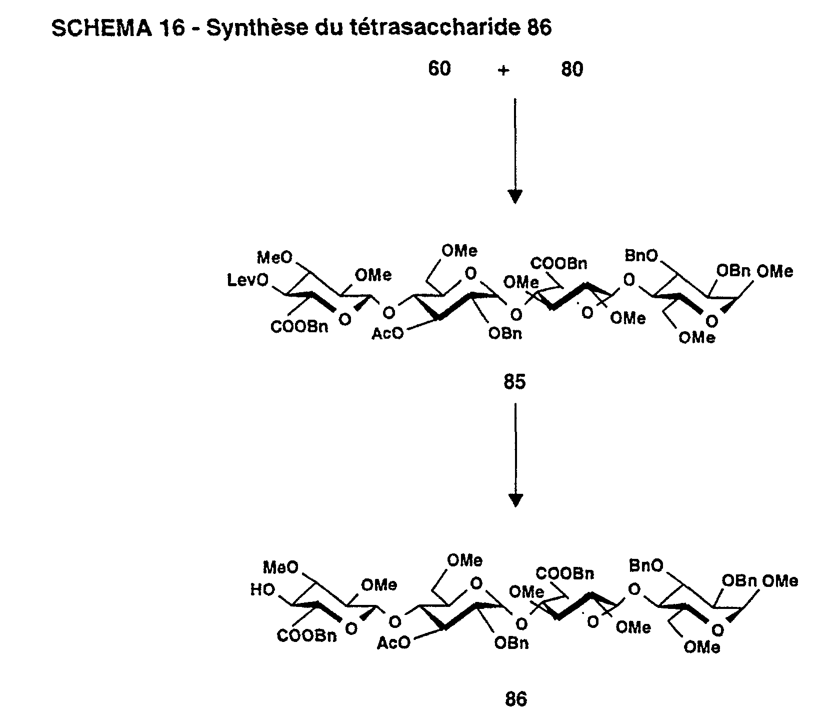

### PRÉPARATION 73

### Méthyl O-(benzyl-4-O-lévulinoyl-2,3-di-O-méthyl-β-D-glucopyranosyluronate)-(1→4)-O-(3-O-acétyl-2-O-benzyl-6-O-méthyl-α-D-glucopyranosyl)-(1→4)-O-(benzyl-2,3-di-O-méthyl-α-L-idopyranosyluronate)-(1→4)-2,3-di-O-benzyl-6-O-méthyl-α-D-glucopyranoside (85).

Un mélange du composé 80 (300 mg) et du composé 60 (455,6 mg) est co-évaporé avec du toluène et dissous dans du dichlorométhane (6 ml) sous atmosphère d'azote. Après addition d'un tamis moléculaire 4Å, le mélange est refroidi à -20°C. Après agitation pendant 20 minutes, on ajoute du trifluorométhanesulfonate de triméthylsilyle (15 mol % par rapport au composé 60). Après 10 minutes, le mélange est neutralisé avec de l'hydrogénocarbonate de sodium aqueux. Après filtration du tamis moléculaire, le filtrat est dilué avec du dichlorométhane, lavé avec de l'eau, concentré et purifié par chromatographie sur colonne de gel de silice pour donner 560 mg du composé 85.

CCM : Rf = 0,50, gel de silice, toluène/acétate d'éthyle 3/7 v/v.

### PRÉPARATION 74

### Méthyl O-(benzyl 2,3-di-O-méthyl-β-D-glucopyranosyluronate)-(1→4)-O-(3-O-acétyl-2-O-benzyl-6-O-méthyl-α-D-glucopyranosyl)-(1→4)-O-(benzyl 2,3-di-O-méthyl-α-L-idopyranosyluronate)-(1→4)-2,3-di-O-benzyl-6-O-méthyl-α-D-glucopyranoside (86).

Le composé 85 (532,6 mg) est dissous dans la pyridine (1,9 ml) et on ajoute à température ambiante un mélange d'acide acétique (2,4 ml), d'hydrate d'hydrazine (0,3 ml) dans la pyridine (1,9 ml). Après agitation pendant 9 minutes, on ajoute du dichlorométhane et de l'eau. La phase organique est séparée et lavée successivement avec de l'acide chlorhydrique 0,1 N, de l'hydrogénocarbonate de sodium aqueux et de l'eau. La phase organique est concentrée et purifiée par chromatographie sur une colonne de gel de silice pour donner 451 mg du composé 86.

CCM : Rf = 0,45, gel de silice, toluène/acétate d'éthyle 3/7 v/v.

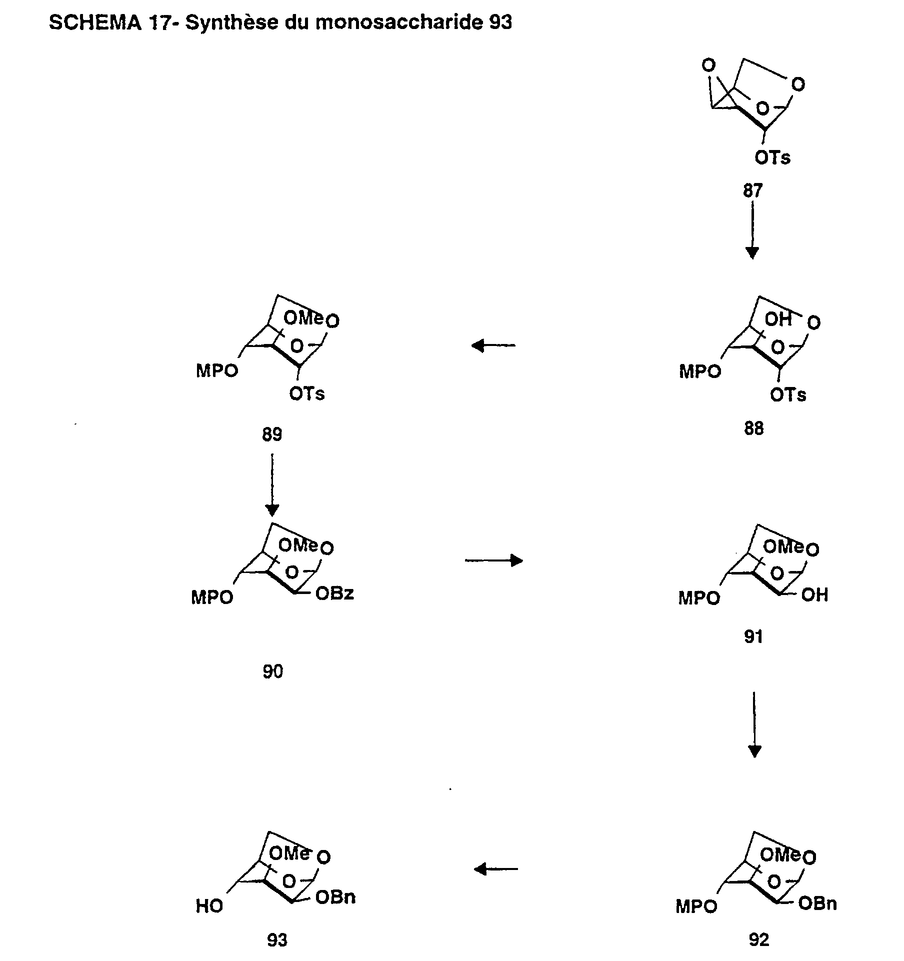

### PRÉPARATION 75

### 1,6-Anhydro-4-O-p-méthoxyphényl-2-O-tosyl-β-D-glucopyranose (88)

Un mélange d'époxyde 87 (20 g, 67 mmol) (M. Cerny *et al*., Collect. Czech. Chem. Commun, 1961, 26, 2542) et de *p*-méthoxyphénol (33,3 g, 268 mmol) sont chauffés sous argon à 90° C. Lorsque le mélange devient liquide, AlCl₃ (0,3 g, 2,35 mmol) est ajouté par petites spatulées puis après 30 minutes d'agitation, et retour à température ambiante, le milieu réactionnel est dilué avec du dichlorométhane (600 ml), neutralisé par de la triéthylamine (0,5 ml), lavé par une solution aqueuse d'hydroxyde de sodium 1M (120 ml), une solution aqueuse saturée de chlorure de sodium (3 x 60 ml), une solution aqueuse d'hydrogénosulfate de potassium à 10% (50 ml), et une solution aqueuse saturée de chlorure de sodium. La phase organique est ensuite séchée (sulfate de sodium), filtrée, et concentrée. Le résidu est purifié par précipitation dans de l'éther et par chromatographie sur silice pour fournir 88 (19,4 g, 69%).

CCM : Rf = 0,37, gel de silice, dichlorométhane/éther éthylique 6/1 v/v

### PRÉPARATION 76

### 1,6-Anhydro-4-O-p-méthoxyphényl-3-O-méthyl-2-O-tosyl-β-D-glucopyranose (89)

A une solution du composé 88 (3,36 g, 7,95 mmol) dans du *N*,*N*-diméthylformamide sec (8 ml) est ajouté de l'iodure de méthyle (54 ml, 954 mmol), de l'oxyde d'argent (18,4 g, 79,5 mmol). Après 16 h d'agitation, le mélange est filtré (Célite), dilué par de l'acétate d'éthyle (300 ml), lavé à l'eau (3 x 100 ml), séché (sulfate de sodium), filtré, concentré, et purifié par chromatographie sur silice pour donner le dérivé 89 (3,23 g, 80%).

CCM : Rf = 0,36, gel de silice, cyclohexane/éther éthylique 2/3 v/v

### PRÉPARATION 77

### 1,6-Anhydro-4-O-p-méthoxyphényl-3-O-méthyl-2-O-benzoyl-β-D-mannopyranose (90)

Le composé 89 (27,4 g, 62,7 mmol) est mis en solution dans du *N*,*N*-diméthylformamide (315 ml). Du benzoate de tétrabutylammonium (341 g, 940 mmol) est ensuite ajouté, et la solution est portée à 150° C pendant 3 h. Après refroidissement à température ambiante; le milieu est dilué à l'acétate d'éthyle (300 ml), lavé à l'eau jusqu'à neutralité, séché, et concentré. Le résidu correspondant au composé 90 est utilisé directement dans l'étape suivante.

CCM : Rf = 0,53, gel de silice, dichlorométhane/éther éthylique 10/1 v/v

### PRÉPARATION 78

### 1,6-Anhydro-4-O-p-méthoxyphényl-3-O-méthyl-β-D-mannopyranose (91)

A une solution du résidu brut 90 précédent dans du dichlorométhane/méthanol (540 ml, 1/1) est ajouté du méthylate de sodium (13,5 g, 250 mmol). Après 1 h d'agitation, le milieu réactionnel est dilué par du dichlorométhane (500 ml), puis lavé par une solution aqueuse à 3% d'acide chlorhydrique et à l'eau. Après séchage, filtration et concentration, le résidu est purifié sur silice pour fournir 91 (10,5 g, 59% en deux étapes).

CCM : Rf = 0,29, gel de silice, toluène/éther éthylique 1/1 v/v

### PRÉPARATION 79

### 1,6-Anhydro-2-O-benzyl-4-O-p-méthoxyphényl-3-O-méthyl-β-D-mannopyranose (92)

A une solution du composé 91 (9,8 g, 34,6 mmol) dans du *N*,*N*-diméthylformamide (180 ml) est ajoutée du bromure de benzyle (6,2 ml, 51,9 mmol), puis à 0° C, de l'hydrure de sodium à 95% (1,16 g, 48,4 mmol). Après 16 h d'agitation, du méthanol (3 ml) est ajouté à 0° C, et le mélange réactionnel est dilué par de l'acétate d'éthyle (200 ml), lavé à l'eau, séché, filtré et concentré. Le résidu correspondant au composé 92 est utilisé directement dans l'étape suivante sans purification. Une fraction analytique est toutefois obtenue après chromatographie sur silice.
[α]²⁰_{D} = -49 (c = 0,73, dichlorométhane).

### PRÉPARATION 80

### 1,6-Anhydro-2-O-benzyl-3-O-méthyl-β-D-mannopyranose (93)

Le composé 92 brut précédent est mis en solution dans un mélange THF/eau (311 ml, 17/1), puis du nitrate de cérium et d'ammonium (76 g, 138 mmol) est ajouté à 0° C. Après 30 minutes d'agitation, le mélange réactionnel est dilué par du dichlorométhane (1l), puis lavé par une solution aqueuse d'hydrogénocarbonate de sodium à 2%, puis à l'eau. Après séchage, filtration et concentration, le résidu est purifié sur silice pour conduire au composé 93 (6,63 g, 72% en deux étapes).
[α]²⁰_{D} = -68 (c = 1,02, dichlorométhane).

La préparation du nonasaccharide 94 ci-dessous est réalisée à partir du monosaccharide 93 selon une séquence de réactions analogue à celle réalisée pour le nonasaccharide 24 à partir du monosaccharide 3.

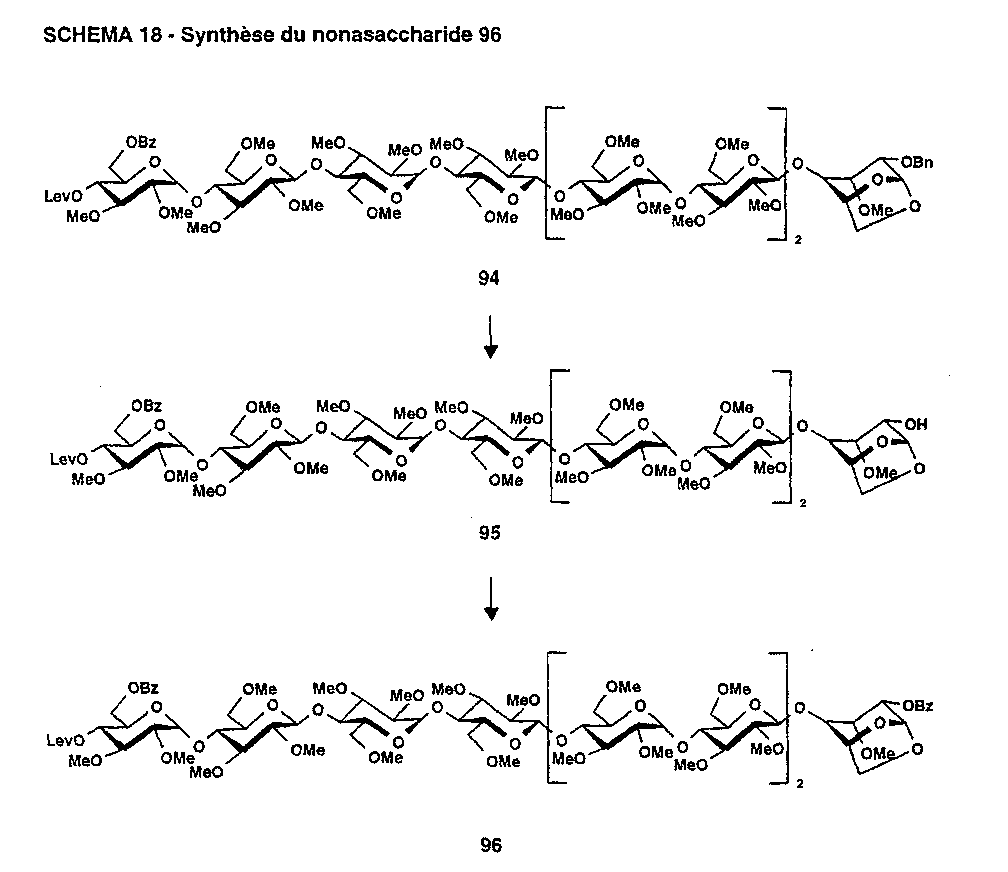

### PRÉPARATION 81

### O-(6-O-Benzoyl-4-O-lévulinoyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-1,6-anhydro-3-O-méthyl-β-D-mannopyranose (95)

A une solution du composé 94 (759 mg, 0,36 mmol) dans un mélange dichlorométhane/*t*-butanol (3,3 ml, 1/2) est ajouté du Pd/C 5% (109 mg). Après 16 heures d'agitation sous 10 bar à 55 °C, le mélange est filtré, et concentré. Le composé 95 est engagé dans l'étape suivante sans purification ni caractérisation.

### PRÉPARATION 82

### O-(6-O-Benzoyl-4-O-lévulinoyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-1,6-anhydro-2-O-benzoyl-3-O-méthyl-β-D-mannopyranose (96)

Du chlorure de benzoyle (63 µl, 0,54 mmol) et de la diméthylaminopyridine (9 mg, 0,073 mmol) sont ajoutés à une solution du composé 95 (726 mg, 0,36 mmol) dans la pyridine anhydre (5,4 ml). Après 2 heures d'agitation à 60 °C, la même quantité de chlorure de benzoyle est ajoutée, et après 35 minutes, la solution est concentrée, le résidu est repris par du dichlorométhane, lavé par une solution aqueuse d'hydrogénosulfate de potassium à 10%, une solution aqueuse d'hydrogénocarbonate de sodium à 2%, puis à l'eau. La phase organique est ensuite séchée sur sulfate de sodium, filtrée, puis concentrée. Le résidu est ensuite purifié sur silice pour donner le composé 96 (684 mg, 90% en 2 étapes).

CCM : Rf = 0,5, gel de silice, toluène/acétone 1/1 v/v

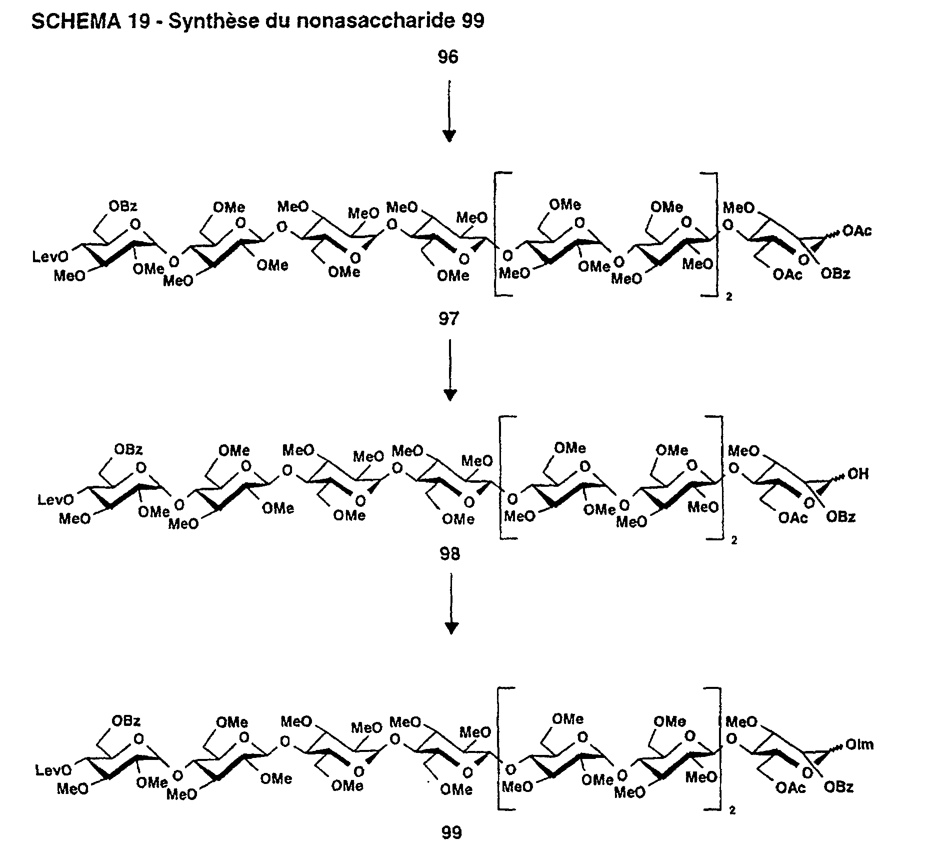

### PRÉPARATION 83

### O-(6-O-Benzoyl-4-O-lévulinoyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-1,6-di-O-acétyl-2-O-benzoyl-3-O-méthyl-α,β-D-mannopyranose (97)

Le composé 96 (657 mg, 0,31 mmol) est traité par un mélange d'anhydride acétique (4,5 ml), d'acide acétique (100 µl) et d'acide trifluoroacétique (0,19 ml) comme pour la préparation 23. Après chromatographie sur une colonne de gel de silice, on obtient le composé 97 (432 mg, 92%).

CCM : Rf = 0,53, gel de silice, toluène/acétone 1/1 v/v

### PRÉPARATION 84

### O-(6-O-Benzoyl-4-O-lévulinoyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-6-O-acétyl-2-O-benzoyl-3-O-méthyl-α,β-D-mannopyranose (98)

Une solution du composé 97 (382 mg, 0,17 mmol) et de benzylamine (736 µl, 6,74 mmol) dans du THF (6 ml) est agitée pendant 5 heures. Le mélange est ensuite versé sur de la glace, et après extraction avec de l'éther éthylique, la phase organique est successivement lavée avec de l'acide chlorhydrique 3% et de l'eau, séchée et concentrée. Après chromatographie sur une colonne de gel de silice, le composé 98 (241 mg) est obtenu.

CCM : Rf = 0,37, gel de silice, toluène/acétate d'éthyle/éthanol 6/2/2 v/v/v

### PRÉPARATION 85

### O-(6-O-Benzoyl-4-O-lévulinoyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-6-O-acétyl-2-O-benzoyl-3-O-méthyl-α,β-D-mannopyranose trichloroacétimidate (99)

Du trichloroacétonitrile (57 µl, 0,56 mmol) et du carbonate de césium (58 mg) sont ajoutés à une solution du composé 98 (241 mg) dans du dichlorométhane (2,2 ml), Après agitation pendant 1 heure, le mélange est filtré, concentré et le résidu est soumis à une chromatographie sur une colonne de gel de silice pour donner l'imidate 99 (221 mg, 56% en deux étapes)

CCM : Rf = 0,19, gel de silice, cyclohexane/acétone 3/2 v/v

### EXEMPLE 1

### Méthyl O-(2,3,4,6-tétra-O-sulfo-α-D-glucopyranosyl)-(1→4)-O-(2,3,6-tri-O-sulfo-α-D-glucopyranosyl)-(1→4)-O-(2,3,6-tri-O-sulfo-β-D-glucopyranosyl)-(1→4)-O-(2,3-di-O-méthyl-6-O-sulfo-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-O-(2,3-di-O-méthyl-6-O-sulfo-α-D-glucopyranosyl)-(1→4)-O-(acide 2,3-di-O-méthyl-β-D-glucopyranosyluronique)-(1→4)-O-(2,3,6-tri-O-sulfo-α-D-glucopyranosyl)-(1→4)-O-(acide 2,3-di-O-méthyl-α-L-idopyranosyluronique)-(1→4)-3-O-méthyl-2,6-di-O-sulfo-α-D-glucopyranoside, sel de sodium (I)

L'hexadécasaccharide 46 (34 mg, 11 µmol) est dissous dans du *N*,*N*-diméthylformamide (1 ml). Le complexe trioxyde de sulfure triéthylamine (180 mg, 0,89 mmol) est ajouté, et le mélange est agité pendant 20 heures à 55° C. On ajoute du complexe triéthylamine/trioxyde de soufre (5mol/mol fonction hydroxyle) à une solution dans le *N*,*N*-diméthylformamide (5 mg/ml) du composé à sulfater. Après un jour à 55°C on dépose la solution au sommet d'une colonne de Sephadex® G-25 (1,6 x 115 cm) éluée par du chlorure de sodium 0,2 M. On concentre les fractions contenant le produit et on dessale en utilisant la même colonne éluée par l'eau. Le composé I (47 mg, 87%) est obtenu après lyophilisation comme poudre blanche. [α]²⁰_{D} = +65 (c = 1,0, eau).

### EXEMPLE 2

### Méthyl O-(2,3,4,6-tétra-O-sulfo-α-D-glucopyranosyl)-(1→4)-O-(2,3,6-tri-O-sulfo-α-D-glucopyranosyl)-(1→4)-O-(2,3,6-tri-O-sulfo-β-D-glucopyranosyl)-(1→4)-O-(2,3-di-O-méthyl-6-O-sulfo-α-D-glucopyranosyl)-(1→4)-O-(2,3,6-tri-O-méthyl-β-D-gluco pyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-O-(2,3-di-O-méthyl-6-O-sulfo-α-D-gluco pyranosyl)-(1→4)-O-(acide 2,3-di-O-méthyl-β-D-glucopyranosyluronique)-(1→4)-O-(6-O-méthyl-2,3-di-O-sulfo-α-D-glucopyranosyl)-(1→4)-O-(acide 2,3-di-O-méthyl-α-L-idopyranosyluronique)-(1→4)-3,6-di-O-méthyl-2-O-sulfo-α-D-glucopyranoside, sel de sodium (II)

### EXEMPLE 3

### Méthyl O-(2,3,4,6-tétra-O-sulfo-α-D-glucopyranosyl)-(1→4)-O-(2,3,6-tri-O-sulfo-α-D-giucopyranosyl)-(1→4)-O-(2,3,6-tri-O-sulfo-β-D-glucopyranosyl)-(1→4)-O-(2,3-di-O-méthyl-6-O-sulfo-α-D-glucopyranosyl)-(1→4)-O-(2,3,6-tri-O-méthyl-β-D-gluco pyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-O-(3-O-méthyl-2,6-di-O-sulfo-α-D-manno pyranosyl)-(1→4)-O-(acide 2,3-di-O-méthyl-β-D-glucopyranosyluronique)-(1→4)-O-(6-O-méthyl-2,3-di-O-sulfo-α-D-glucopyranosyl)-(1→4)-O-(acide 2,3-di-O-méthyl-α-L-idopyranosyluronique)-(1→4)-6-O-méthyl-2,3-di-O-sulfo-α-D-glucopyranoside, sel de sodium (III)

### EXEMPLE 4

### Méthyl O-(2,3,4,6-tétra-O-sulfo-α-D-glucopyranosyl)-(1→4)-O-(2,3,6-tri-O-sulfo-α-D-glucopyranosyl)-(1→4)-O-(2,3,6-tri-O-sulfo-β-D-glucopyranosyl)-(1→4)-O-(2,3-di-O-méthyl-6-O-sulfo-α-D-glucopyranosyl)-(1→4)-O-(2,3,6-tri-O-méthyl-β-D-gluco pyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-O-(3-O-méthyl-2,6-di-O-sulfo-α-D-manno pyranosyl)-(1→4)-O-(acide 2,3-di-O-méthyl-β-D-glucopyranosyluronique)-(1→4)-O-(6-O-méthyl-2,3-di-O-sulfo-α-D-glucopyranosyl)-(1→4)-O-(acide 2,3-di-O-méthyl-α-L-idopyranosyluronique)-(1→4)-3,6-di-O-méthyl-2-O-sulfo-α-D-glucopyranoside, sel de sodium (IV)

### EXEMPLE 5

### Méthyl O-(2,3,4,6-tétra-O-sulfo-α-D-glucopyranosyl)-(1→4)-O-(2,3,6-tri-O-sulfo-α-D-glucopyranosyl)-(1→4)-O-(2,3,6-tri-O-sulfo-β-D-glucopyranosyl)-(1→4)-O-(2,3-di-O-méthyl-6-O-sulfo-α-D-glucopyranosyl)-(1→4)-O-(2,3,6-tri-O-méthyl-β-D-glucopyranosyl)-(1→4)-[O-(2,3,6-tri-O-méthyl-a-D-glucopyranosyl)-(1→4)-O-(2,3,6-tri-O-méthyl-β-D-glucopyranosyl)-(1→4)]₃-O-(2,3-di-O-méthyl-6-O-sulfo-α-D-gluco pyranosyl)-(1→4)-O-(acide 2,3-di-O-méthyl-β-D-glucopyranosyluronique)-(1→4)-O-(6-O-méthyl-2,3-di-O-sulfo-α-D-glucopyranosyl)-(1→4)-O-(acide 3-O-méthyl-2-O-sulfo-a-L-idopyranosyluronique)-(1→4)-3,6-di-O-méthyl-2-O-sulfo-α-D-glucopyranoside, sel de sodium (V)

Les composés des exemples 2 à 5 ont été préparés de façon analogue par couplage d'un tétrasaccharide approprié au nonasaccharide approprié, puis couplage avec le trisaccharide 41 ou 43 (ainsi que décrit aux préparations 36 ou 38), suivi des réactions habituelles de déprotection et sulfatation.

Dans le cas de l'exemple 2, le tétrasaccharide utilisé est celui décrit à la préparation 55 (composé 62), et le nonasaccharide utilisé est celui décrit à la préparation 25 (composé 27).

Dans le cas de l'exemple 3, le tétrasaccharide utilisé est celui décrit à la préparation 74 (composé 86), et le nonasaccharide utilisé est celui décrit à la préparation 85 (composé 99).

Dans le cas de l'exemple 4, le tétrasaccharide utilisé est celui décrit à la préparation 55 (composé 62), et le nonasaccharide utilisé est celui décrit à la préparation 85 (composé 99).

Dans le cas de l'exemple 5, le tétrasaccharide utilisé est le composé 68, et le nonasaccharide utilisé est celui décrit à la préparation 25 (composé 27).

**TABLEAU I**

| | | | | |
|---|---|---|---|---|
| ¹H-RMN (D₂O, 4,80 ppm) δ de protons H-1 (ppm) : | | | | |
| Unité 1 = unité à l'extrémité réductrice | | | | |
| Unité 16 = unité à l'extrémité non réductrice | | | | |

| Unité | EXEMPLE 2 | EXEMPLE 3 | EXEMPLE 4 | EXEMPLE 5 |
|---|---|---|---|---|
| 1 | 5,08 | 4,68 | 5,07 | 5,08 |
| 2 | 4,93 | 5,02 | 5,02 | 4,93 |
| 3 | 5,37 | 5,41 | 5,43 | 5,37 |
| 5 | 5,49 | 5,57 | 5,48 | 5,57 |
| 6 | 4,66 | 4,71 | 4,65 | 4,70 |
| 7,9,11 | 5,67 | 5,67 | 5,68 | 5,67 |
| 4,8,10,12 | 4,39-4,49 | 4,39-4,49 | 4,37-4,47 | 4,39-4,39 |
| 13 | 5,61 | 5,61 | 5,60 | 5,61 |
| 14 | 4,8 | 4,94 | 4,93 | 4,93 |
| 15 | 5,59 | 5,59 | 5,59 | 5,59 |
| 16 | 5,69 | 5,69 | 5,69 | 5,68 |

**TABEAU II**

| Masse méthode "negative ion electron spray ionisation" (ESI) | | | |
|---|---|---|---|
| EXEMPLE | formule | calculée | trouvée |
| 2 | C₁₂₉H₂₂₄O₁₂₈S₁₅ | 4304,1 | 4304,5 |
| 3 | C₁₂₇H₂₂₀O₁₃₄S₁₇ | 4436,2 | 4436,2 |
| 4 | C₁₂₈H₂₂₂O₁₃₁S₁₆ | 4370,1 | 4370,4 |
| 5 | C₁₂₈H₂₂₂O₁₃₁S₁₆ | 4370,1 | 4370,5 |

## Revendications

1. Polysaccharide de synthèse sous forme acide et ses sels pharmaceutiquement acceptables, dont la forme anionique répond à l'une des formules (I) à (V) suivantes :

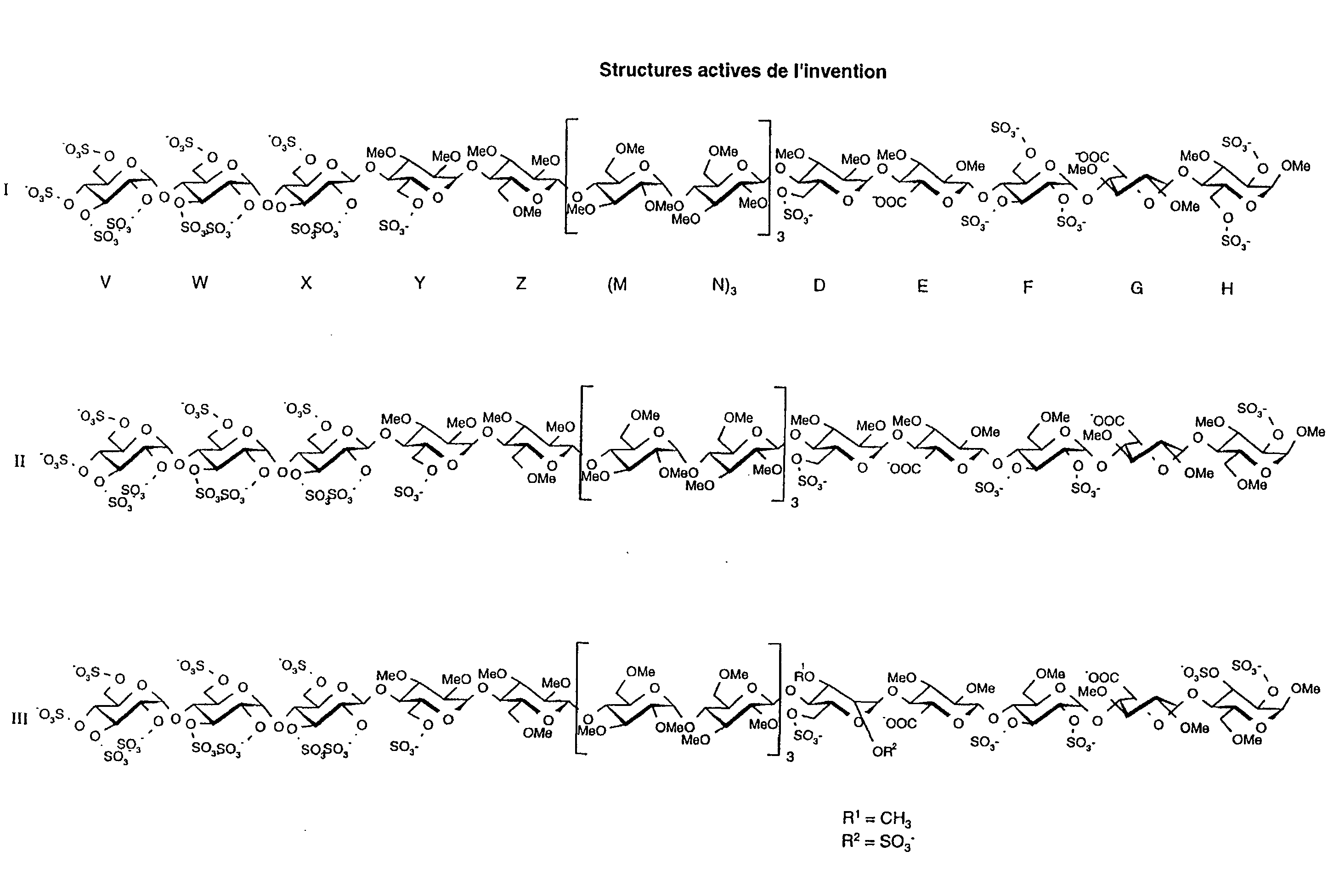

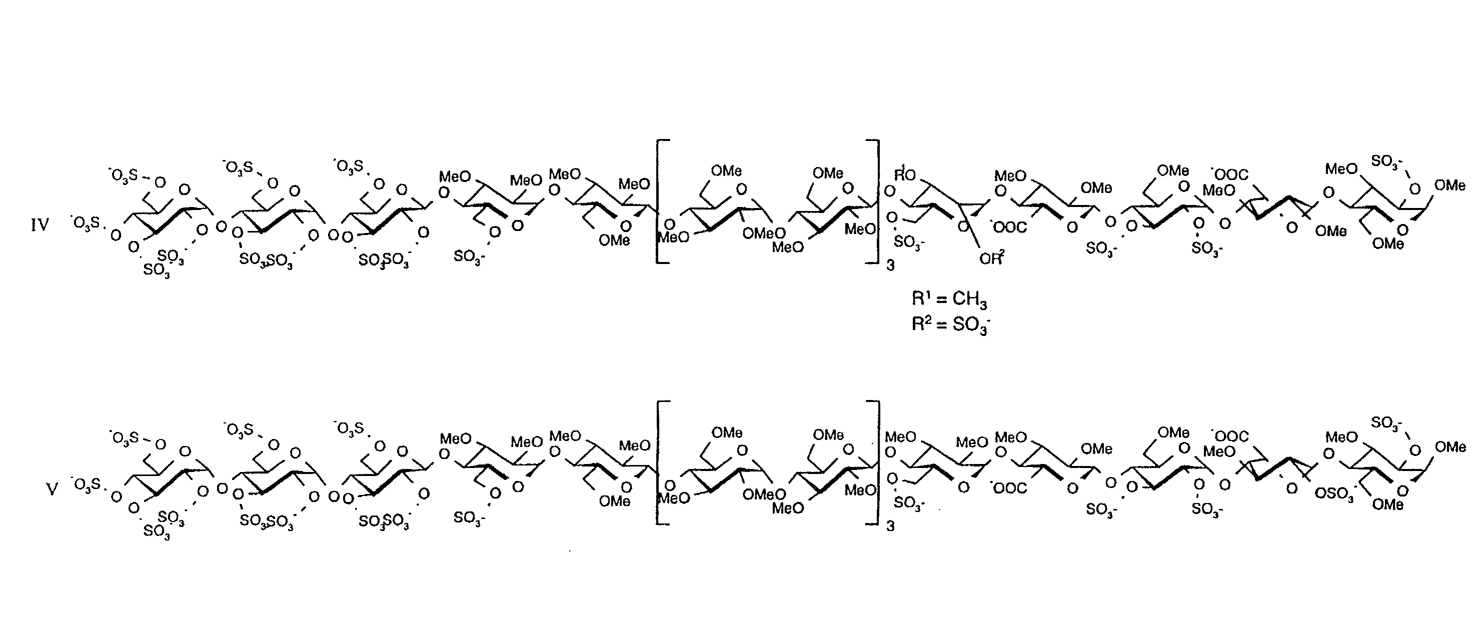

2. Polysaccharide selon la revendication 1 sous la forme de sel de sodium ou de potassium.

3. Polysaccharide selon l'une quelconque des revendications 1 et 2 de formule :
Méthyl *O*-(2,3,4,6-tétra-*O*-sulfo-α-D-glucopyranosyl)-(1→4)-*O*-(2,3,6-tri-*O*-sulfo-α-D-glucopyranosyl)-(1→4)-*O*-(2,3,6-tri-*O*-sulfo-β-D-glucopyranosyl)-(1→4)-*O*-(2,3-di-*O* -méthyl-6-*O*-sulfo-α-D-glucopyranosyl)-(1→4)-*O*-(2,3,6-tri-*O*-méthyl-β-D-glucopyranosyl)-(1→4)-[*O*-(2,3,6-tri-*O*-méthyl-α-D-glucopyranosyl)-(1→4)-*O*-(2,3,6-tri-O-méthyl-β-D-gluco pyranosyl)-(1→4)]₃-*O*-(2,3-di-*O*-méthyl-6-*O*-sulfo-6-α-D-glucopyranosyl)-(1→4)-*O*-(acide 2,3-di-*O*-méthyl-β-D-glucopyranosyluronique)-(1→4)-*O*-(2,3,6-tri-*O*-sulfo-α-D-gluco pyranosyl)-(1→4)-*O*-(acide 2,3-di-*O*-méthyl-α-L-idopyranosyluronique)-(1→4)-3-*O*-méthyl-2,6-di-*O*-sulfo-α-D-glucopyranoside, sel de sodium.

4. Compositions pharmaceutiques contenant comme principe actif un polysaccharide selon l'une quelconque des revendications 1 à 3 sous forme de sel avec une base pharmaceutiquement acceptable ou sous forme acide, en association ou en mélange avec un excipient inerte, non toxique, pharmaceutiquement acceptable.

5. Composition pharmaceutique selon la revendication 4, sous forme d'unités de dosage, dans laquelle le principe actif est mélangé à au moins un excipient pharmaceutique.

6. Composition selon la revendication 5 dans laquelle chaque unité de dosage contient de 0,1 à 100 mg de principe actif.

7. Composition selon la revendication 6 dans laquelle chaque unité de dosage contient de 0,5 à 50 mg de principe actif.

8. Utilisation d'un polysaccharide selon les revendications 1 à 3 pour la préparation d'un médicament utile dans les pathologies dépendantes d'un dysfonctionnement de la coagulation.

9. Utilisation d'un polysaccharide selon les revendications 1 à 3 pour la préparation d'un médicament utile dans le traitement et la prévention des troubles du système cardio-vasculaire et cérébro-vasculaire, les troubles thromboemboliques associés à l'athérosclérose et au diabète, ou les troubles thromboemboliques associés à la rethrombose après thrombolyse, à l'infarctus, à la démence d'origine ischémique, aux maladies artérielles périphériques, à l'hémodialyse, aux fibrillations auriculaires ou encore lors de l'utilisation de prothèses vasculaires de pontages aorto-coronariens.

10. Utilisation selon la revendication 9 **caractérisée en ce que** les médicaments préparés sont utilisés dans la prévention ou le traitement des maladies thromboemboliques.

11. Utilisation selon la revendication 10 **caractérisée en ce que** les médicaments préparés sont utilisés dans la prévention ou le traitement de l'angine instable, de l'attaque cérébrale, la resténose après angioplastie, l'endartérectomie, ou la pose de prothèses endovasculaires.

## Patentansprüche

1. Synthetisches Polysaccharid in Form der Säure und dessen pharmazeutisch annehmbare Salze, deren anionische Form einer der folgenden Formeln (I) bis (V) entspricht:

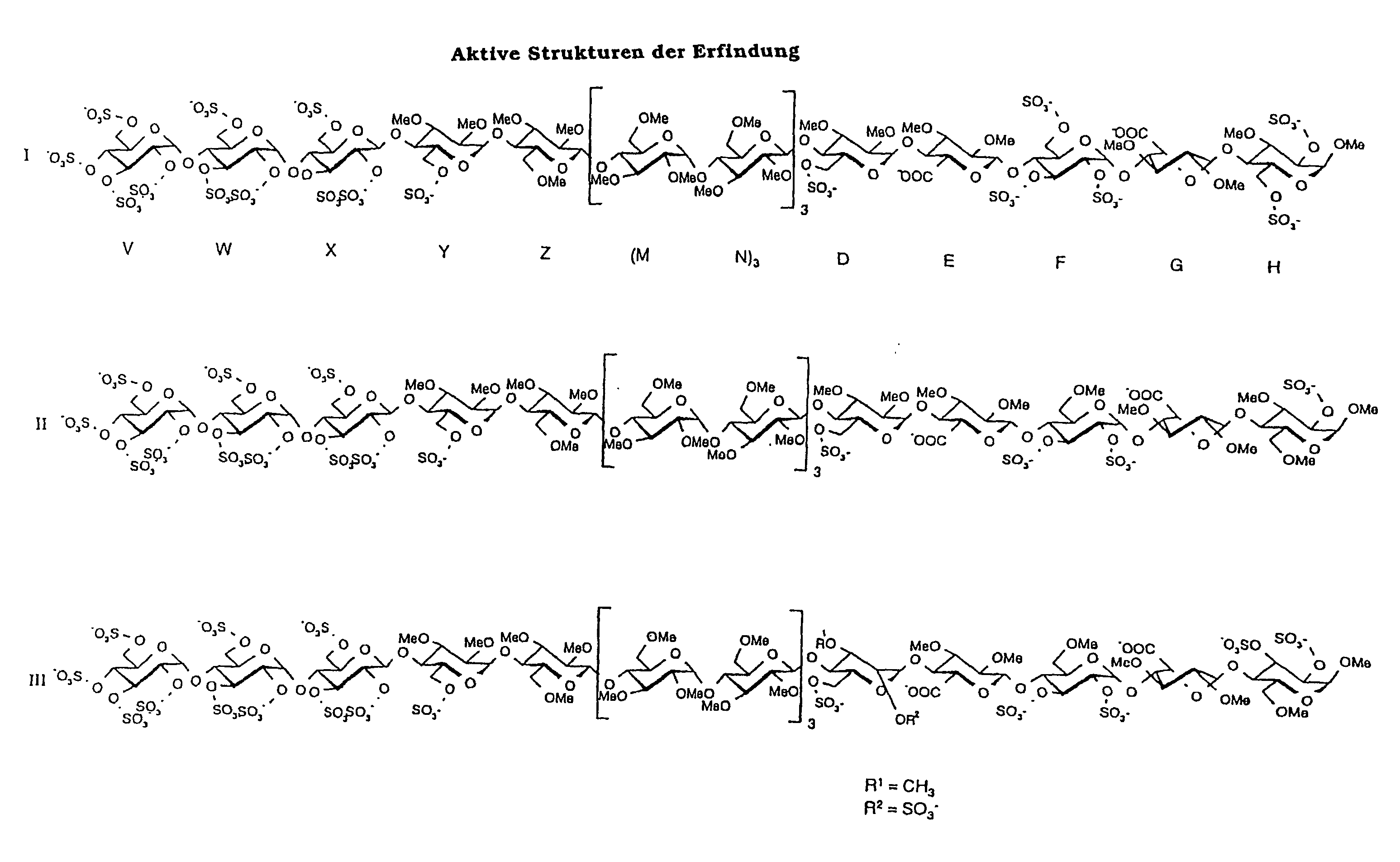

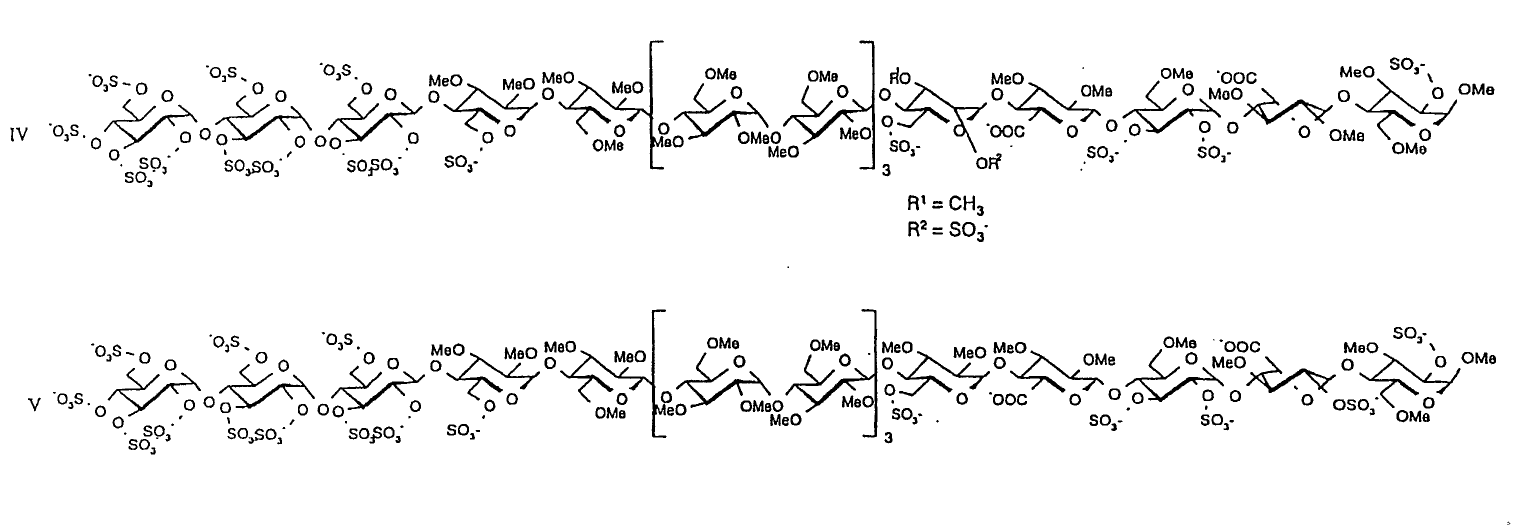

2. Polysaccharid nach Anspruch 1 in Form des Natriumsalzes oder des Kaliumsalzes.

3. Polysaccharid nach einem der Ansprüche 1 und 2 der Formel:
Methyl-*O*-(2,3,4,6-tetra-*O*-sulfo-α-D-glucopyranosyl)-(1→4)-*O*-(2,3,6-tri-*O*-sulfo-α-D-glucopyranosyl)-(1→4)-*O*-(2,3,6-tri-*O*-sulfo-β-D-glucopyranosyl)-(1→4)-*O*-[2,3-di-*O*-methyl-6-*O*-sulfo-α-D-glucopyranosyl)-(1→4)-*O*-(2,3,6-tri-*O*-methyl-β-D-glucopyranosyl)-(1→4)-[*O*-(2,3,6-tri-*O*-methyl-α-D-glucopyranosyl)-(1→4)-*O*-(2,3,6-tri-*O*-methyl-β-D-glucopyranosyl)-(1→4)]₃-*O*-(2,3-di-*O*-methyl-6-*O*-sulfo-α-D-glucopyranosyl)-(1→4)-*O*-(2,3-di-*O*-methyl-β-D-glucopyranosyluronsäure)-(1→4)-*O*-(2,3-6-tri-*O*-sulfo-α-D-glucopyranosyl)-(1→4)-*O*-(2,3-di-*O*-methyl-α-L-idopyranosyluronsäure)-(1→4)-3-*O*-methyl-2,6-di-*O*-sulfo-α-D-glucopyranosid, Natriumsalz.

4. Pharmazeutische Zubereitungen enthaltend als Wirkstoff ein Polysaccharid nach einem der Ansprüche 1 bis 3 in Form des Salzes mit einer pharmazeutisch annehmbaren Base oder in Form der Säure, in Kombination oder in Mischung mit einem inerten, nichttoxischen, pharmazeutisch annehmbaren Trägermaterial.

5. Pharmazeutische Zubereitung nach Anspruch 4 in Form von Dosiseinheiten, in denen der Wirkstoff mit mindestens einem pharmazeutischen Trägermaterial vermischt ist.

6. Zubereitung nach Anspruch 5, worin jede Dosiseinheit 0,1 bis 100 mg des Wirkstoffs enthält.

7. Zubereitung nach Anspruch 6, worin jede Dosiseinheit 0,5 bis 50 mg des Wirkstoffs enthält.

8. Verwendung eines Polysaccharids nach einem der Ansprüche 1 bis 3 für die Herstellung eines Arzneimittels zur Behandlung von pathologischen Zuständen, die von einer Dysfunktion der Blutgerinnung abhängen.

9. Verwendung eines Polysaccharids nach den Ansprüchen 1 bis 3 für die Herstellung eines Arzneimittels zur Behandlung und Vorbeugung von Störungen des kardiovaskulären und zerebro-vaskulären Systems, von thromboembolischen Störungen, die mit Atherosklerose und Diabetes verknüpft sind, oder von thromboembolischen Störungen, die mit Rethrombose nach Thrombolyse, Infarkten, Dementien ischämischen Ursprungs, peripheren arteriellen Erkrankungen, der Hämodialyse, Herzmuskelflimmern oder Bypass-Gefäßprothesen verbunden sind.

10. Verwendung nach Anspruch 9, **dadurch gekennzeichnet, daß** die hergestellten Arzneimittel bei der Vorbeugung oder der Behandlung von thromboembolischen Krankheiten eingesetzt werden.

11. Verwendung nach Anspruch 10, **dadurch gekennzeichnet, daß** die hergestellten Arzneimittel bei der Vorbeugung oder der Behandlung von instabiler Angina, Zerebralvorfällen, Restenosen nach der Angioplastie, der Endarterektomie oder dem Anbringen von endovaskulären Prothesen verwendet werden.

## Claims

1. Synthetic polysaccharide in the form of the acid and its pharmaceutically acceptable salts, the anionic form of which corresponds to one of the following formulae (I) to (V):

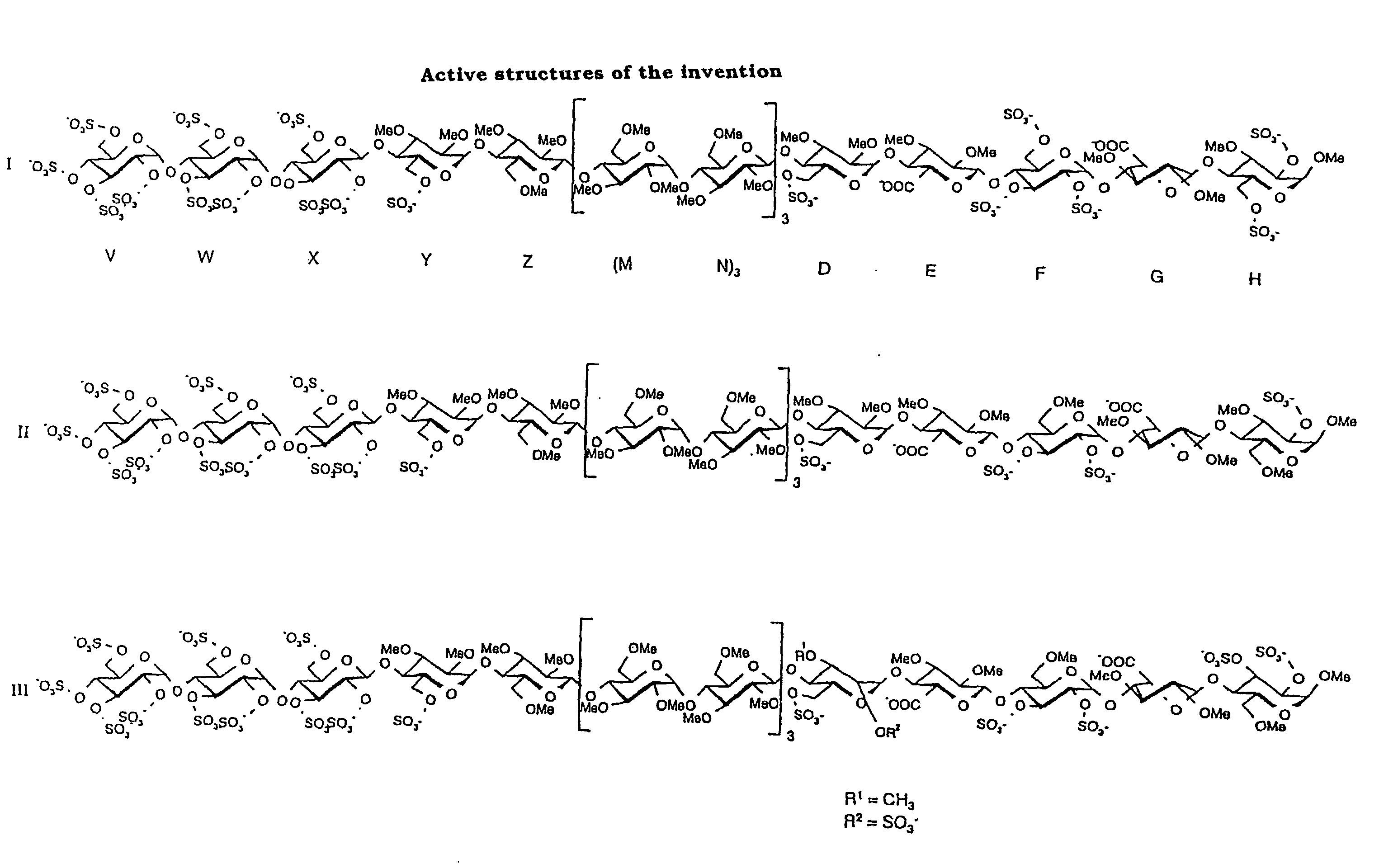

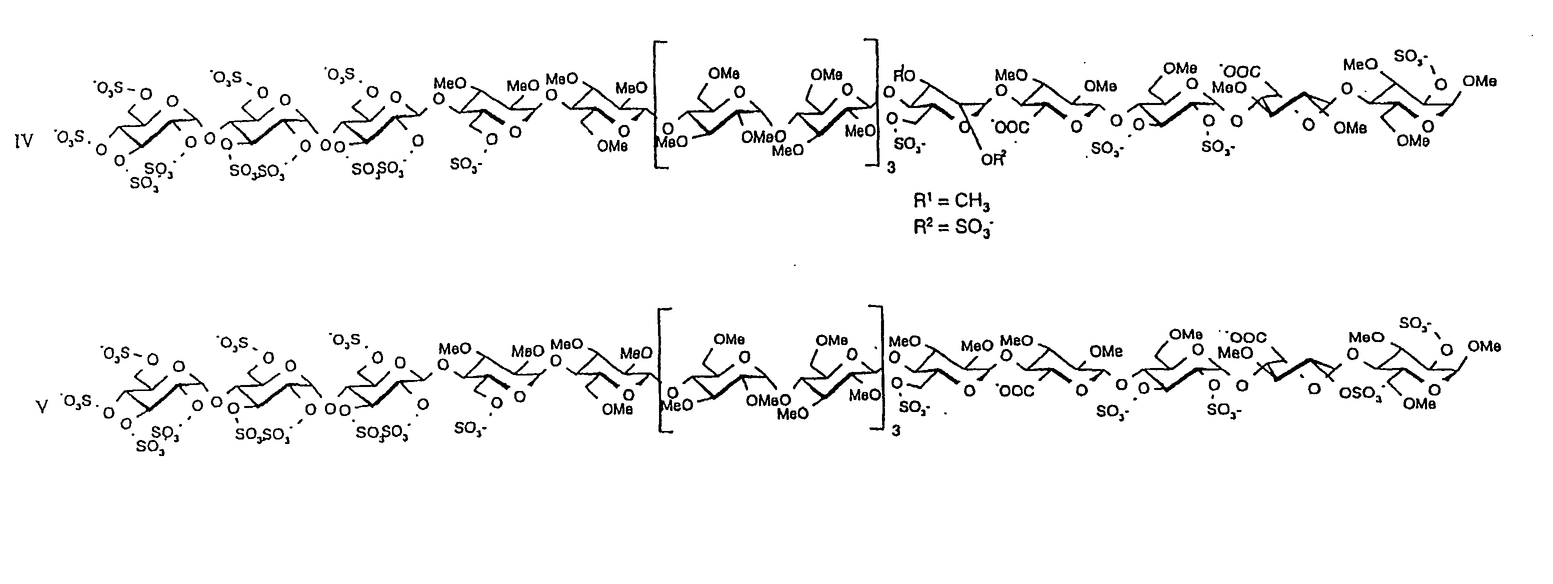

2. Polysaccharide according to claim 1 in the form of the sodium or potassium salt.

3. Polysaccharide according to any one of claims 1 and 2 of the formula:
methyl-*O*-(2,3,4,6-tetra-*O*-sulfo-α-D-glucopyranosyl)-(1→4)-*O*-(2,3,6-tri-*O*-sulfo-α-D-glucopyranosyl)-(1→4)-*O*-(2,3,6-tri-*O*-sulfo-β-D-glucopyranosyl)-(1→4)-*O*-(2,3-di-*O*-methyl-6-*O*-sulfo-α-D-glucopyranosyl)-(1→4)-*O*-(2,3,6-tri-*O*-methyl-β-D-glucopyranosyl)-(1→4)-[*O*-(2,3,6-tri-*O*-methyl-a-D-glucopyranosyl)-(1→4)-O-(2,3,6-tri-*O*-methyl-β-D-glucopyranosyl)-(1→4)]₃-*O*-(2,3-di-*O*-methyl-6-*O*-sulfo-α-D-glucopyranosyl)-(1→4)-*O*-(2,3-di-*O*-methyl-β-D-glucopyranosyluronic acid)-(1→4)-*O*-(2,3-6-tri-*O*-sulfo-α-D-glucopyranosyl)-(1→4)-*O*-(2,3-di-*O*-methyl-α-L-idopyranosyluronic acid)-(1→4)-3-*O*-methyl-2,6-di-*O*-sulfo-α-D-glucopyranoside, sodium salt.

4. Pharmaceutical compositions comprising as an active agent a polysaccharide according to any one of claims 1 to 3 in the form of the salt with a pharmaceutically acceptable salt or in the form of the acid, in association or in mixture with inert, non-toxical pharmaceutically acceptable excipient.

5. Pharmaceutical composition according to claim 4 in the form of dosage units, wherein the active agent is mixed with at least one pharmaceutical excipient.

6. Composition according to claim 5, wherein each dosage unit contains 0.1 to 100 mg of the active ingredient.

7. Composition according to claim 6, wherein each dosage unit contains 0.5 to 50 mg of the active ingredient.

8. The use of a polysaccharide according to claims 1 to 3 for the manufacture of a medicament useful for pathologies depending from a dysfunctioning of the coagulation.

9. The use of a polysaccharide according to the claims 1 to 3 for the manufacture of a medicament for the treatment and the prevention of cardio-vascular and cerebro-vascular troubles, thromboembolic troubles associated with atherosclerosis and diabetes, or thromboembolic troubles associated to rethrombosis after thrombolysis, infarctus, dementia of ischemical origin, peripheral arterial diseases, haemoddialysis, auricular fibrillations or the use of vascular protheses of aorta-coronary bypasses.

10. The use according to claim 9, **characterized in that** the prepared medicaments are used in the prevention or the treatment of thrombo-embolic diseases.

11. The use according to claim 10, **characterized in that** the manufactured medicaments are used in the prevention or the treatment of instable angina, cerebral attacks, restenose after angioplastia, endarterectomy or deposition of endovasculary protheses.
